# EUROPEAN PATENT APPLICATION

(11) **EP 3 269 699 A1**
(43) Date of publication of application: **17.01.2018**
(21) Application number: 16382336.2
(22) Date of filing: 15.07.2016
(51) Int. Cl.: C07C 51/15, C07C 53/126, C07C 53/128, C07C 53/134, C07C 57/30, C07C 61/08, C07C 67/29, C07C 67/313, C07C 69/24, C07C 69/34, C07C 69/46, C07C 69/92

(54) **CATALYTIC CARBOXYLATION OF ACTIVATED ALKANES AND/OR OLEFINS**

(71) Applicant: Fundació Institut Català d'investigació Quimica, 43007 Tarragona (ES); ICREA, Institució Catalana de Recerca I Estudis Avançats, 08010 Barcelona (ES)
(72) Inventor: MARTÍN ROMO, Rubén, 08029 Barcelona (ES); JULIÁ HERNÁNDEZ, Francisco, 43002 Tarragona (ES); CORNELLA, Josep, 43717 La Bisbal del Penedès, Tarragona (ES)
(74) Representative: Hoffmann Eitle

(57) **Abstract**

The present invention relates to a method of reacting starting materials with an activating group, namely alkanes carrying a leaving group and/or olefins, with carbon dioxide under transition metal catalysis to give carboxyl group-containing products. It is a special feature of the method of the present invention that the carboxylation predominantly takes place at a preferred position of the molecule irrespective of the position of the activating group. The carboxylation position is either an aliphatic terminus of the molecule or it is a carbon atom adjacent to a carbon carrying an electron withdrawing group. The course of the reaction can be controlled by appropriately choosing the reaction conditions to yield the desired regioisomer.

## Description

### Field of the invention

The present invention relates to a method of reacting activated alkanes and/or olefins with carbon dioxide under transition metal catalysis to give carboxyl group-containing products.

### Background of the invention

Carbon dioxide is abundantly available in the atmosphere and exists as by-product in processes such as the burning of fossil fuels. It is therefore inexpensive, available in large amounts and since it is also non-flammable and hence easy to handle, it has been recognized to an increasing extent as an attractive chemical reagent, for instance as a synthon for carboxylic acid groups.

In addition to traditional methods of introducing a carboxylic acid group using carbon dioxide as a reagent (such as the reaction of Grignard compounds, alkyl- or aryl-lithium compounds with carbon dioxide), metal-catalyzed carboxylation protocols have been developed which are advantageous because the reaction conditions are usually relatively mild such that other functional groups are stable and do not have a negative effect on the reaction.

Kirillov et al. in Dalton Trans., 2015, 44, pages 16212-162223 review the preparation of carboxylic acid derivatives by catalytic carboxylation of unsaturated hydrocarbons using either a different Ni(II) based system or a different substrate range.

Beller et al. in Nature Communications, January 20, 2015, 6, pages 1-15 (article no. 5933) review the use of carbon dioxide as a building block in organic synthesis.

Among the metal-catalyzed carboxylation protocols, Nickel-catalysts have gained particular attention.

Martin et al. in J. Am. Chem. Soc. 2013, 135, pages 1221-1224 report on the nickel-catalyzed carboxylation of benzyl halides with carbon dioxide.

Martin et al. in J. Am. Chem. Soc. 2014, 136, pages 11212-11215 report on the nickel-catalyzed carboxylation of primary alkyl bromides and sulfonates with carbon dioxide in which the corresponding primary carboxylic acid reaction products were formed.

Martin et al. in J. Am. Chem. Soc. 2014, 136, pages 1062-1069 report on the nickel-catalyzed carboxylation of carbon-oxygen bonds with carbon dioxide, wherein the carbon atom of the carbon-oxygen bond is an aryl ring atom or a benzyl carbon atom and the oxygen atom is the oxygen atom of a carboxylic ester moiety.

Martin et. al. in J. Am. Chem. Soc. 2014, 136, pages 17702-17705 report on the nickel-catalyzed carboxylation of allyl esters with carbon dioxide. The catalyst is formed from nickel(II) halide compounds in the presence of manganese or zinc as a reducing agent and a bipyridine, terpyridine or phenanthroline as a ligand. The reaction proceeds in a regiodivergent manner, i.e. it depends on the ligand employed in the catalyst on which carbon atom of the allyl moiety the carboxyl group is introduced.

It would be desirable to provide a protocol for the metal-catalyzed carboxylation of further starting materials of different structure. It would also be desirable to provide such a protocol for the metal-catalyzed carboxylation that is improved in that the efficiency of the reaction is increased and/or that the reaction proceeds in a regioconvergent manner. It is an object of the present invention to solve these technical problems.

The process of the present invention allows using different starting materials selected from two general classes of substances including alkenes and secondary or tertiary bromoalkanes and related substances. It is even possible to make use of such starting materials that are not activated by other functional groups. In one aspect, the present invention therefore solves the further problem of providing a method for direct carboxylation, which can be carried out using a considerable variety of starting materials. In fact, the present invention describes the first method to obtain aliphatic saturated carboxylic acids via direct carboxylation of unactivated olefins, in which CO₂ directly participates in the carboxylation step.

Yet another benefit of the process of the present invention is its regioconvergent nature, i.e. the possibility of obtaining a single end product having a carboxyl group attached to a well-defined position even if starting materials with different structures or a mixture of such starting materials is used. There is therefore less need to purify the starting material. The present invention thus allows obtaining the desired target molecule with reduced efforts. Hence, according to this aspect, the present invention solves the problem of providing a method for direct carboxylation that involves reduced purification efforts.

### Summary of the Invention

The aforementioned technical problems are solved by the invention disclosed in the following, namely by a method as defined in the claims. That is, the present invention is directed to a method of direct carboxylation of a compound represented by formula (III) 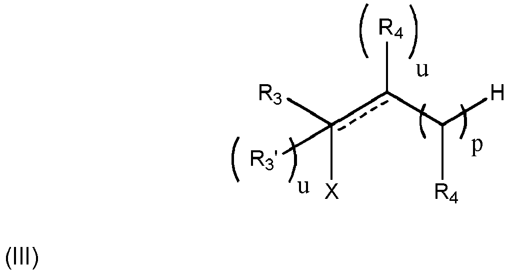 wherein
p is an integer from 0 to 15;
u is 0 or 1;
each R₄ represents a radical independently selected from the group consisting of hydrogen,
   a linear or branched C₁₋₂₀ alkyl group optionally carrying one or more substituents selected from fluoro and chloro,
   a linear or branched C₁₋₂₀ alkyloxy group;
X represents hydrogen, a bromine atom, an iodine atom, a phenylsulfonate wherein the phenyl ring is optionally substituted with a methyl group, a methylsulfonate and a trifluoromethylsulfonate;
   with the proviso that when X represents hydrogen, then the bond represented by the combination of a solid line and a dotted line is a double bond, and u is 0; and that when X is not hydrogen, then the bond represented by the combination of a solid line and a dotted line is a single bond, and u is 1;
R₃ and R₃' are each independently selected from hydrogen and a radical of formula -Y(-Z)ₙ,
wherein
Y is selected from a linear C₁₋₂₀ alkyl, a branched C₃₋₂₀ alkyl and a C₃₋₈ cycloalkyl;
n is an integer from 0 to the number of carbon atoms in Y;
each Z is independently selected from the group consisting of:
   fluoro, chloro,
a radical of formula -OR₅, a radical of formula -C(O)R₅, a radical of formula-OC(O)R₅, a radical of formula -C(O)OR₅, a radical of formula -NHC(O)R₅, a radical of formula -C(O)NHR₅, a radical of formula -NHC(O)OR₅, a radical of formula -OC(O)NHR₅, R₅ being selected from the group consisting of a linear or branched C₁₋₂₀ alkyl groups, and a cyclic group comprising 1 ring or 2 to 5 condensed rings,
   wherein each ring has 5 or 6 members selected from the group consisting of C, CH, CH₂, O, S, N and NR₆,
      wherein R₆ is selected from hydrogen and C₁₋₆alkyl, each ring being independently unsaturated, saturated or aromatic; and each ring being further optionally substituted with one or more radicals selected from the group consisting of: fluoro, chloro, C₁-₆alkyl, C₁-₆haloalkyl, C₁-₆alkyloxy, C₁-₆alkylcarbonyl, C₁-₆alkylcarbonyloxy, C₁-₆alkylcarbonylamino, and C₁-₆alkylaminocarbonyl,
an aldehyde group,
nitro,
cyano,
a group of formula -SO₂R₁₁ wherein R₁₁ is selected from C₁-₆alkyl, C₁-₆haloalkyl, phenyl and phenyl substituted with one or more C₁-₆alkyl groups; and
a cyclic group comprising 1 ring or 2 to 5 condensed rings, wherein each ring has 5 or 6 members selected from the group consisting of C, CH, CH₂, O, S, N and NR₆,
   wherein R₆ is selected from hydrogen and C₁-₆alkyl, each ring being independently unsaturated, saturated or aromatic;
      and each ring being further optionally substituted with one or more radicals selected from the group consisting of: C₁-₆alkyl, C₁-₆haloalkyl, C₁-₆alkyloxy, C₁-₆alkylcarbonyl, C₁-₆alkylcarbonyloxy, C₁-₆alkylcarbonylamino, C₁-₆alkylaminocarbonyl, -Rₐ-R_{b},
   wherein Rₐ is a single bond or a diradical selected from -O-, -S-, - C(=O)-, -C(=O)-O-, -O-C(=O)-, -C(=O)-NH-, -NH-C(=O)-, -O-C(=O)-NH-, -NH-C(=O)-O-, -O-C(=O)-O-, -S(O)₂-, -S(O)₂-NH-,-NH-S(O)₂-, -N=N-, -NH-C(=O)-NH-,
      and
   wherein R_{b} is selected from a C₆₋₁₀ aryl group, a C₃₋₈ cycloalkyl group optionally containing one or two double bonds, or a saturated or unsaturated heterocyclic group containing 5 to 7 ring members wherein each ring member is selected from C, CH, CH₂, O, S, N and NR₆, wherein R₆ is selected from hydrogen and C₁-₆alkyl;
or; alternatively, R₃ and R₃' together with the carbon atom to which they are attached form a cyclic group comprising 1 ring or 2 to 5 condensed rings, wherein each ring has 5 or 6 members selected from the group consisting of C, CH, CH₂, O, S, N and NR₆, wherein R₆ is selected from hydrogen and C₁₋₆alkyl, each ring being independently unsaturated, or saturated; and each ring being further optionally substituted with one or more radicals selected from the group consisting of: C₁-₆alkyl, C₁-₆haloalkyl, C₁-₆alkyloxy, C₁-₆alkylcarbonyl, C₁-₆alkylcarbonyloxy, C₁-₆alkylcarbonylamino, C₁-₆alkylaminocarbonyl, -Rₐ-R_{b},
   wherein Rₐ is a single bond or a diradical selected from -O-, -S-, - C(=O)-, -C(=O)-O-, -O-C(=O)-, -C(=O)-NH-, -NH-C(=O)-, -O-C(=O)-NH-, -NH-C(=O)-O-, -O-C(=O)-O-, -S(O)₂-, -S(O)₂-NH-,-NH-S(O)₂-, -N=N-, -NH-C(=O)-NH-,
   and wherein R_{b} is selected from a C₆₋₁₀ aryl group, a C₃₋₈ cycloalkyl group optionally containing one or two double bonds, or a saturated or unsaturated heterocyclic group containing 5 to 7 ring members wherein each ring member is selected from C, CH, CH₂, O, S, N and NR₆, wherein R₆ is selected from hydrogen and C₁-₆alkyl;
with the proviso that R₃ is not hydrogen if X is not hydrogen.

Preferred embodiments become apparent from the following description and the attached dependent claims.

### Detailed Description of the invention

### Definitions and general Remarks

Unless specified otherwise, all amount indications are provided on a molar basis, with absolute indications being provided in the unit of moles and relative indications being provided as mol% with the total of the respective composition being 100 mol%.

Unless specified otherwise, all indications of point values are intended to allow variation of ±10%, preferably of ±5% and most preferably ±0% of the specified value around the point value.

Unless specified otherwise, the present application uses the term "radical" to characterize an atomic group that is monovalent and that is bonded to the remainder of the molecule via a covalent single bond. Correspondingly, the present application uses the term "diradical" to characterize an atomic group that is divalent and thus binds to two other moieties via two covalent bonds. The term "substituent" is also used herein to have the same meaning as the term radical.

Unless specified otherwise, the term "comprising" is intended to permit the additional presence of unspecified further components or measures. It is however a preferred embodiment that no further components or measures are present, i.e. the term "comprising" encompasses also the meaning "consisting of" as a preferred embodiment.

The term "aryl" is defined in the context of the present disclosure as a carbocyclic aromatic ring or a system of 2 to 5 condensed carbocyclic rings wherein at least one of these condensed rings is aromatic. It is further conceived that the aryl group may carry one or more additional aromatic carbocyclic rings being attached to the primary aromatic ring (or condensed ring system) via covalent single bonds, provided that the total number of carbocyclic rings does not exceed five. Examples include, in a non-limitative way, phenyl, naphthyl, indenyl, phenanthrenyl, anthracenyl, biphenylyl, perylenyl, and phenylnaphthalenyl. The aryl group may be unsubstituted or be further substituted (in addition to any aryl substituents bonded to the primary aryl ring (or condensed ring system) mentioned above).

Unless specified otherwise, the term "polar aprotic solvent" is intended to refer to a solvent that has a dielectric constant above 25.0 and/or a dipole moment above 2.5 D and which does not release any protons under the conditions of the method of the present invention. Typical examples are N-methylpyrrolidone, THF, ethyl acetate, acetone, DMF, acetonitrile, dimethyl sulfoxide, HMPT, and propylene carbonate.

Unless specified otherwise, the term "catalytically effective amount" is intended to characterize an amount of the respective catalytically active substance, which gives rise to at least one of an increase in yield or reaction rate of at least 10% in comparison with a corresponding reaction being carried out in the absence of the catalytically active substance.

The expression "secondary" in relation to a carbon atom means that the carbon atom is attached to two bonding partners other than hydrogen. A "secondary carbon" as an internal member of a carbon chain is a carbon atom that is bonded to two carbon atom chain members as well as two hydrogen atoms. The expression "secondary position" is used to characterize the position of a group of interest (e.g. a leaving group) to characterize that the particular group of interest is attached to a carbon atom that is connected to two adjacent carbon atoms and one hydrogen atom and thus a carbon atom which is neither a terminal carbon atom nor a carbon atom that has three further bonding partners (other than hydrogen). Similarly, a "quaternary" carbon atom refers to a carbon atom which is substituted with 4 groups different from hydrogen. Similarly, a "tertiary" carbon atom refers to a carbon atom which is substituted with 3 groups different from hydrogen and is further substituted with 1 hydrogen atom.

Unless specified otherwise, all specified atoms and groups of atoms are intended to have saturated valences. Hence, if an atom has more valences than bonding partners shown or apparent from the context, all remaining valences are meant to be saturated with hydrogen atoms. Similarly, if different atoms or groups of atoms are listed as alternatives for the formation of different moieties, it is intended that a selection is to be made only from those atoms and atom groups, which offer a sufficient number of free valences for incorporation into the target moiety. For instance, if there is a reference to CH and CH₂ as members of saturated or aromatic rings, it should be understood that the CH₂ ring member is available only for the saturated ring option but not the aromatic ring option.

Unless it is apparent from the context that a different meaning is intended, all terms and expressions used herein are to be understood as having their generally accepted meaning, as represented by standard chemistry textbooks and encyclopedias such as Clayden, Jonathan; Greeves, Nick; Warren, Stuart; Wothers, Peter (2001); Organic Chemistry (1st ed.), Oxford University Press, ISBN 978-0-19-850346-0.

Unless specified otherwise, references to the method of the present invention are to be understood as references to the direct carboxylation reaction described below. This method optionally includes the preparation of a nickel catalyst, which can either be done in situ or separately. However, unless specified otherwise, references to the conditions of the method or reaction of the invention (e.g. regarding solvent or temperature) are to be understood as references to the conditions of the direct carboxylation reaction forming the core of the invention - and not as references to the nickel catalyst formation reaction, if carried out separately.

Unless specified otherwise, the direct carboxylation reaction of the present invention may yield the product not only in the free acid form, but also in the form of a salt. Consequently, all references to reaction compounds carrying a group -COOH are to be understood as encompassing also a reference to the group -COOM, wherein M represents a counter-ion. This can be any organic or inorganic counter-ion including in particular ammonium ions such as NR₄⁺ with each R being independently hydrogen or an alkyl group of 1-4 carbon atoms, alkali ions such as Na⁺ and K⁺ as well as alkaline earth ions such as Mg²⁺ and Ca²⁺ or other transition metals such as Mn²⁺ or Zn²⁺. Of course, if an alkaline earth ion or other ion with a multiple positive charge is used, the stoichiometry must be adjusted to ensure that the resulting product is not electrically charged. That is, if the counter-ion carries n positive charges, 1/n moles of counter-ion are present per mole of carboxyl group.

Unless expressly specified otherwise, all references to carbon-carbon double bonds are intended to characterize both cis (Z) and trans (E) geometries. This applies even to cases where a structural formula is shown that may suggest a particular cis (Z) or trans (E) geometry when applying standard conventions for drawing chemical formulae.

In the context of the present invention, the term "activating group" is used to characterize a carbon-carbon double bond or a leaving group X, wherein X is as defined below but with X being other than hydrogen.

In the context of the present invention, the term "electron withdrawing group" characterizes a group that withdraws electron density from the atom to which the group is attached. In aromatic systems, electron withdrawing groups commonly have a Hammett constant σ (para) value higher than 0.25. Suitable electron withdrawing groups in the context of the invention are selected from the group consisting of a C₁-₆ alkylcarbonyl, a C₁-₆alkyloxycarbonyl, a C₁-₆alkylaminocarbonyl, an aldehyde group, nitro, cyano, sulfones, sulfoxides and sulfonates.

In the context of the present invention, when the carboxylation may occur at one or more atoms in the compound of formula(III), the term "kinetic control reaction conditions" characterizes conditions of reaction where the fastest carboxylation process is predominant. In other words, these are conditions yielding predominantly the same product as the product obtained at lowest possible temperature at which the reaction of the present invention can be carried out.

In the context of the present invention, when the carboxylation may occur at one or more atoms in the compound of formula (III), the term "thermodynamic control reaction conditions" characterizes conditions where the most stable carboxylation product is predominantly obtained. In other words, these are conditions yielding predominantly the same product as the product obtained at highest possible temperature at which the reaction of the present invention can be carried out.

### Overview

The present invention provides a method for direct carboxylation of specific starting materials. Said starting materials are compounds with an aliphatic chain that is activated by a leaving group or by a carbon-carbon double bond.

More specifically, the starting materials can be characterized by the following general formulae:

R₃-CH(X)-CH₂-(CHR₄)ₚ-H (III')

R₃-CH=CH-(CHR₄)ₚ-H (III")

R₃-C(R₃')(X)-CH(R₄)-(CHR₄)ₚ-H (III"')

The meanings of variable groups R₃, R₃', R₄, of p and of leaving group X are defined herein above and in more detail below.

The course of the reaction and the resulting product of the direct carboxylation reaction are determined by the specific structure of the starting material and, to some extent, by the reaction conditions.

### (i) Impact of structure of starting material

The direct carboxylation of the present invention normally yields primary carboxylic acids, i.e. carboxylic acids wherein the carboxyl group is attached to a carbon atom that is bonded via a single bond to the remainder of the molecule. Said primary carboxylic acids are formed by attaching the carboxyl group to a terminal aliphatic carbon atom of the starting material. The method of the present invention thus extends the carbon chain of the starting material by creation of a new carbon-carbon bond.

The activating group (i.e. the leaving group X or the double bond) not being located at a terminal position of the starting material, the course of the reaction can be explained by a "chain walking mechanism". To allow such a chain walking mechanism, it is a prerequisite that each member of the carbon chain between the activating group and the terminal carbon atom carries at least one hydrogen atom. Consequently, it is a prerequisite that the compound of formula (III) does not comprise any quaternary carbon atom between the carbon atom bearing the X group or double bond and the carbon atom to which the carboxyl group is attached in the product of the reaction.

If the starting material contains more than one terminal aliphatic carbon atom fulfilling the above requirement for chain walking, direct carboxylation normally could in theory take place in different positions. However, in practice, direct carboxylation normally takes place predominantly at the terminal carbon atom that is closest to the activating group.

Hence, if the starting material contains susceptible terminal aliphatic carbon atoms as part of R₃ and/or R₄, the following alternative reaction products can be envisaged, depending on the question which of the termini has the shortest chain to the activating group:

R₃-CH₂₋ᵤ(R₃')ᵤ-CH₂₋ᵤ(R₄)ᵤ-(CHR₄)ₚ-COOH (IIa)

HOOC-R₃-CH₂₋ᵤ(R₃')ᵤ-CH₂₋ᵤ(R₄)ᵤ-(CHR₄)ₚ-H (IIb)

R₃-CH₂₋ᵤ(R₃')ᵤ-CH₂₋ᵤ(R₄)ᵤ-(CHR₄ₐ)_{q}-CH(R_{4b}(COOH))-(CHR_{4c})ᵣ-H (IIc)

wherein u is 0 or 1 and preferably 0, R₃ and R₄ have the meanings specified herein above and below, and wherein each of R₄ₐ, R_{4b} and R_{4c} independently has the same meaning as specified for R₄, but with the proviso that R_{4b} has a terminal aliphatic carbon atom, which is connected with the activating group (i.e. leaving group X or double bond) via a chain of secondary and tertiary carbon atoms that is not interrupted by any quaternary carbon atoms;
wherein the product of formula (IIa) can only be formed if the terminal carbon atom of the chain -(CHR₄)ₚ- is indeed a terminal carbon atom, which means that the group R₄ attached to the carbon atom of the chain -(CHR₄)ₚ-, which is furthest away from the activating group, must not be an alkyl group. Products of formula (IIa) are preferably formed using starting materials, wherein the last carbon atom of the chain -(CHR₄)ₚ- carries a hydrogen atom as group R₄, such that the chain -(CHR₄)ₚ- becomes a -(CHR₄)ₚ₋₁-CH₂- chain. Otherwise, the terminus of this aliphatic chain would be found in group R₄, so that the resulting product would be of the type of formula (IIc) (with r=0).
wherein q = 0 - 7, r = 0 - 14 and q + r + 1 = 1 - 15;
and wherein in formula (IIb), the carboxyl group is attached to a carbon atom of R₃ that is a primary aliphatic carbon atom in the starting material, which is connected with the activating group (i.e. leaving group X or double bond) via a chain of secondary and tertiary carbon atoms that is not interrupted by any quaternary carbon atoms;
and wherein in formula (IIc), the carboxyl group is attached to the above-mentioned terminal aliphatic carbon atom of the R_{4b} group that is a primary aliphatic carbon atom in the starting material, and that is connected with the activating group (i.e. leaving group X or double bond) via a chain of secondary and tertiary carbon atoms that is not interrupted by any quaternary carbon atoms.

In preferred embodiments, in the compound of formula (III), u=0. Therefore, the compounds of formulae (IIa), (IIb) and (IIc) are simplified to the compounds of formulae (IIa'), (IIb') and (IIc') respectively:

R₃-CH₂-CH₂-(CHR₄)ₚ-COOH (IIa')

HOOC-R₃-CH₂-CH₂-(CHR₄)ₚ-H (IIb')

R₃-CH₂-CH₂-(CHR₄ₐ)_{q}-CH(R_{4b}(COOH))-(CHR_{4c})ᵣ-H (IIc')

Apart from the length of the carbon chain, the course of the reaction may further be influenced by electronic effects of further substituents that may be present. This is illustrated in sub-section (ii) below.

### (ii) Impact of reaction conditions - kinetic versus thermodynamic control

Different reaction products may be obtained if the starting material contains a substituent group that has an electron withdrawing effect and is especially one of the following groups:
a radical of formula -C(O)R₅,
a radical of formula -C(O)OR₅,
a radical of formula -C(O)NHR₅ R₅ being as defined above
an aldehyde group,
cyano,
nitro, and
a group of formula -SO₂R₁₁
   wherein R₁₁ is selected from C₁-₆alkyl, C₁-₆haloalkyl, phenyl and phenyl substituted with one or more C₁-₆alkyl groups.

For such starting materials, the course of the reaction depends not only on the chain lengths mentioned above, but also on the reaction conditions. It is also a prerequisite that the compound of formula (III) does not comprise any quaternary carbon atom between the carbon atom bearing the X group and the carbon atom to which the carboxyl group is attached in the product of the reaction. In particular, different reaction products are obtained depending whether conditions are chosen such that the reaction is thermodynamically controlled (e.g. high reaction temperature, fast reaction) or kinetically controlled (e.g. low temperature, slow reaction).

Specifically, kinetic control leads to direct carboxylation of a terminal aliphatic carbon atom as described under item (i) above.

Conversely, thermodynamic control leads to direct carboxylation of the carbon atom adjacent to the above electron withdrawing group, which provides the most stable (i.e. energetically favoured) product.

The situation can be illustrated by the following example reaction, which is based on Example 5 below:

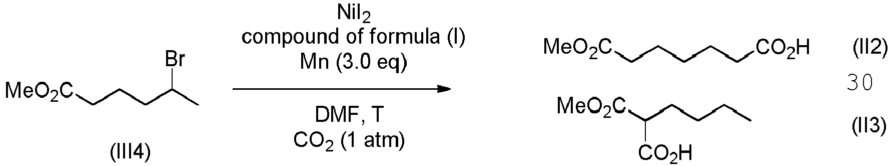

At high temperatures (i.e. under thermodynamic reaction control conditions), the carbon atom adjacent to the electron withdrawing group (methyl ester group) is carboxylated (product (II3)), whereas the primary aliphatic carbon atom at the opposite terminus is carboxylated at low temperatures (i.e. under kinetic reaction control conditions, product (II2)).

Hence, in addition to the reaction products (IIa), (IIb) and (IIc) above, it is also conceivable for some starting materials to control reaction conditions such that the following thermodynamic reaction control product (IId) is obtained:

(Z-)ₙY(COOH)-CH₂₋ᵤ(R₃')ᵤ-CH₂₋ᵤ(R₄)ᵤ-(CHR₄)ₚ-H (IId)

wherein R₃ in this structural formula is represented by (Z-)ₙY, and wherein the carboxyl group is attached to the carbon atom of group Y that is adjacent to the carbon atom carrying a substituent group Z that is an electron withdrawing group as described above, and wherein R₄, n, u and p are as defined above.

In a preferred embodiment, u = 0, so that formula (IId) is simplified to

(Z-)ₙY(COOH)-CH₂-CH₂-(CHR₄)ₚ-H (IId')

Again, if different carbon atoms are available for direct carboxylation via thermodynamic control because they have adjacent carbons carrying an electron withdrawing group Z, the reaction proceeds in practice such that predominantly the carbon atom is carboxylated, which provides the most stable (i.e. energetically favoured) product.

A thermodynamic reaction control product similar to the product (IId) above can also be obtained if at least one R₄ group of the starting material contains three F and/or Cl groups bonded to the same carbon atom and at least one of the adjacent carbon atoms carries at least one hydrogen atom and is connected with the activating group via a chain of secondary and/or tertiary groups that is not interrupted by any quaternary carbon atoms. The combined electron-withdrawing effect of these halogen atoms may also direct the direct carboxylation reaction to the adjacent carbon atom if suitable thermodynamic control conditions are chosen for the reaction. The resulting product can be characterized by the following formula (IIe):

R₃-CH₂₋ᵤ(R₃')ᵤ-CH₂₋ᵤ(R₄)ᵤ-(CHR₄ₐ)_{q}-CH(R_{4d}(COOH))-(CHR_{4c})ᵣ-H (IIe)

wherein R₃, R₄, R₄ₐ, R_{4c}, u, q and r are as defined above and wherein R_{4d} is a group as defined for R₄ above, but with the proviso that the group contains three atoms selected from F and Cl bonded to the same carbon atom and that the -COOH group is bonded to an adjacent carbon atom that is connected with the activating group via a chain of secondary and/or tertiary groups that is not interrupted by any quaternary carbon atoms.

Depending on the synthetic target, all of the above-mentioned products may be valuable. All of the above alternative courses of the direct carboxylation reaction are therefore to be regarded as different variants of the method of the present invention. As indicated above, a variety of different reaction products (IIa) to (IIe) are conceivable for the method of the present invention. The method of the present invention is nevertheless a valuable synthetic tool because - depending on starting material and reaction conditions - only one of these reaction products is predominantly obtained. The above general rules determining the predominant reaction product can be summarized as follows:
A product of **formula (IIa)** is predominantly obtained if all of the following conditions for the compound of formula (III) are fulfilled:
   - The number of carbon atoms between the activating group and the terminal carbon atom of the chain formed by -(CHR₄)ₚ- is smaller than the number of carbon atoms in any other aliphatic carbon atom chain involving R₃ or R₄, and connecting a terminal aliphatic carbon atom with the activating group only via secondary and/or tertiary carbons but without quaternary carbon atoms;
   - the compound of formula (III) does not contain any electron withdrawing groups that are connected with the activating group only via secondary or tertiary aliphatic carbon atoms and/or the reaction is carried out under kinetic control reaction conditions.
A product of **formula (IIb)** is predominantly obtained if all of the following conditions for the compound of formula (III) are fulfilled:
   - The number of carbon atoms between the activating group and the terminal aliphatic carbon atom of R₃ is smaller than the number of carbon atoms in any other aliphatic carbon atom chain involving -(CHR₄)ₚ- and/or individual R₄ groups connecting a terminal aliphatic carbon atom with the activating group only via secondary and/or tertiary carbons but without quaternary carbon atoms;
   - the carbon atom chain connecting the terminal aliphatic carbon atom of R₃ with the activating group includes only secondary and/or tertiary carbon atoms but no quaternary carbon atoms;
   - the compound of formula (III) does not contain any electron withdrawing groups connected with the activating group only via secondary or tertiary aliphatic carbon atoms and/or the reaction is carried out under kinetic control reaction conditions.
A product of **formula (IIc)** is predominantly obtained if all of the following conditions for the compound of formula (III) are fulfilled:
   - The number of carbon atoms between the activating group and the terminal carbon atom of R_{4b} is smaller than the number of carbon atoms in any other aliphatic carbon atom chain involving R₃ or -(CHR₄)ₚ-, connecting a terminal aliphatic carbon atom with the activating group only via secondary and/or tertiary carbons but without quaternary carbon atoms;
   - the carbon atom chain connecting the terminal aliphatic carbon atom of R_{4b} with the activating group includes only secondary and/or tertiary carbons but no quaternary carbon atoms;
   - the compound of formula (III) does not contain any electron withdrawing groups connected with the activating group only via secondary or tertiary aliphatic carbon atoms and/or the reaction is carried out under kinetic control reaction conditions.
A product of **formula (IId)** is predominantly obtained if all of the following conditions for the compound of formula (III) are fulfilled:
   - the starting material contains at least one electron withdrawing group connected with the activating group only via secondary or tertiary aliphatic carbon atoms but without quaternary carbon atoms and
   - the reaction is carried out under thermodynamic control reaction conditions.
A product of **formula (IIe)** is predominantly obtained if all of the following conditions for the compound of formula (III) are fulfilled:
   - at least one R₄ group of the starting material contains three F and/or Cl groups bonded to the same carbon atom and at least one of the adjacent carbon atoms carries at least one hydrogen atom and is connected with the activating group via an aliphatic chain having secondary and/or tertiary carbon atoms and not being interrupted by any quaternary carbon atoms and
   - the reaction is carried out under thermodynamic control reaction conditions.

### (A) The starting material

The method of the present invention can be carried out using two different general types of starting materials, namely aliphatic compounds carrying a leaving group in a secondary or tertiary position, and aliphatic compounds having a carbon-carbon double bond. The leaving group is preferably a bromine atom. However, other leaving groups such as an iodine atom, a tosylate group, mesylate group, triflate group or related groups may also be used. The starting material may be linear, branched, cyclic or any combination thereof. The starting material may preferably have from 2 to 100 carbon atoms, more preferably from 2 to 50 carbon atoms and most preferably from 2 to 20 carbon atoms. The starting material may carry one or more further substituents as long as these substituents do not interfere with the reaction underlying the method of the present invention. A list of suitable substituents is provided for instance in appended claim 1. It should however be clear that further substituents may exist that are also compatible with the method of the present invention.

The position of the leaving goup X is not particularly restricted as long as it is attached to a secondary carbon atom or a tertiary carbon atom that is adjacent to a carbon atom carrying a hydrogen atom. For instance, if the starting material is a linear bromoalkane having a backbone of n carbon atoms, the bromo substituent can be attached to any one of carbon atoms 2, 3, ..., n-1. The same applies, of course, to other leaving groups.

Similarly, if an alkene is used as the starting material, the position of the carbon-carbon double bond is also not particularly restricted. For instance, in the case of a linear alkene with a backbone of n carbon atoms, said double bond can be in anyone of positions 1 and 2, 2 and 3, 3 and 4, ..., (n-1) and n.

Suitable starting materials are especially those of the general formula (III), as described herein above.

Preferred starting materials are those where u is 0, i.e. starting materials wherein general formula (III) falls within the scope of preferred formula (III') as specified herein above.

Preferred starting materials are characterized by the following formula (IIIa): 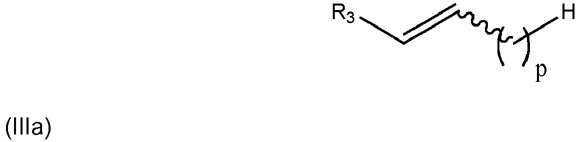 wherein the double bond is in cis or trans configuration and wherein R₃ and p are as specified herein above.

A preferred starting material is that wherein R₃ is a radical selected from the group consisting of:
hydrogen and a radical of formula -Y(-Z)ₙ, wherein
Y is selected from a linear C₁₋₂₀ alkyl group,
a branched C₃₋₂₀ alkyl group, and
and a C₃₋₈ cycloalkyl group,
wherein each Z is independently selected from the group consisting of:
   fluoro, chloro, a radical of formula -OR₅, a radical of formula -OC(O)R₅, a radical of formula -C(O)OR₅, R₅ being selected from the group consisting of a linear or branched C₁₋₂₀ alkyl groups, and a cyclic group comprising 1 ring or 2 to 5 condensed rings,
      wherein each ring has 5 or 6 members selected from the group consisting of C, CH, and CH₂,
      each ring being independently unsaturated, saturated or aromatic;
      and each ring being further optionally substituted with one or more radicals selected from the group consisting of: fluoro, chloro, C₁-₆alkyl, C₁-₆haloalkyl, C₁-₆alkyloxy, C₁-₆alkylcarbonyl, C₁-₆alkylcarbonyloxy, C₁-₆alkylcarbonylamino, and C₁-₆alkylaminocarbonyl,
   a cyclic group comprising 1 ring or 2 to 5 condensed rings,
      wherein each ring has 6 members selected from the group consisting of C, CH, and CH₂,
      each ring being independently unsaturated, saturated or aromatic;
      and each ring being further optionally substituted with one or more radicals selected from the group consisting of: C₁-₆alkyl, C₁-₆haloalkyl, C₁-₆alkyloxy, C₁-₆alkylcarbonyl, C₁-₆alkylcarbonyloxy, C₁-₆alkylcarbonylamino, C₁-₆alkylaminocarbonyl, and -R_{b}, wherein R_{b} is a C₆₋₁₀aryl group.

Another preferred starting material is that wherein R₃ is a radical selected from the group consisting of:
hydrogen and a radical of formula -Y(-Z)ₙ, wherein
Y is selected from a linear C₁₋₂₀ alkyl group,
and a branched C₃₋₂₀ alkyl group, and
a C₃₋₈ cycloalkyl group,
   wherein each Z is independently a radical of formula -OC(O)R₅, a radical of formula -C(O)OR₅, R₅ being selected from the group consisting of a linear or branched C₁₋₂₀ alkyl groups, and a cyclic group comprising 1 ring or 2 condensed aromatic rings,
      wherein each ring has 5 or 6 members selected from the group consisting of C, and CH;
   and each ring being further optionally substituted with one or more radicals selected from the group consisting of: fluoro, C₁-₆alkyl, C₁-₆haloalkyl, and C₁-₆alkyloxy, or a cyclic group comprising 1 ring or 2 to 5 condensed rings, wherein each ring has 6 members selected from the group consisting of C, CH, and CH₂, each ring being independently unsaturated, saturated or aromatic; and each ring being further optionally substituted with one or more radicals selected from the group consisting of: C₁-₆alkyl, C₁-₆haloalkyl, C₁-₆alkyloxy, C₁-₆alkylcarbonyl, C₁-₆alkylcarbonyloxy, C₁-₆alkylcarbonylamino, C₁-₆alkylaminocarbonyl, and -R_{b}, wherein R_{b} is a C₆₋₁₀aryl group.

Another preferred starting material is that wherein R₃ is a radical selected from the group consisting of:
hydrogen and a radical of formula -Y(-Z)ₙ, wherein
Y is selected from
a linear C₁₋₂₀ alkyl group,
and a branched C₃₋₂₀ alkyl group,
   wherein each Z is independently a radical of formula -OC(O)R₅, a radical of formula -C(O)OR₅, R₅ being selected from the group consisting of methyl, and phenyl optionally substituted with one or more radicals selected from the group consisting of: fluoro, and C₁-₆alkyloxy, or a phenyl ring optionally substituted with one or more radicals selected from the group consisting of: C₁-₆alkyl, C₁-₆haloalkyl, C₁-₆alkyloxy, C₁-₆alkylcarbonyl, C₁-₆alkylcarbonyloxy, C₁-₆alkylcarbonylamino, C₁-₆alkylaminocarbonyl, and -R_{b}, wherein R_{b} is a C₆₋₁₀ aryl group.

Another preferred starting material is that wherein R₃ is a C₁₋₁₀ alkyl group optionally substituted with a phenyl ring, the phenyl ring being optionally substituted with a *tert-*butylcarbonyloxy, or a radical of formula -OC(O)R₅, a radical of formula -C(O)OR₅, R₅ being selected from the group consisting of methyl, and phenyl optionally substituted with one or more radicals selected from the group consisting of: fluoro, and methyloxy.

Another preferred starting material is characterized by the following formula (IIIb): 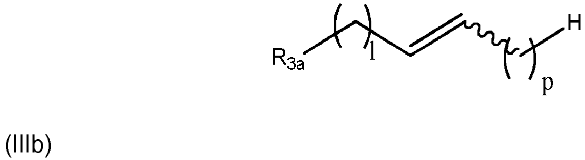 wherein
p represents an integer of 0 to 3;
I represents an integer of 0 to 3;
p + I is less than or equal to 3;
R₃ₐ represents a radical selected from a linear or branched C₁₋₍₂₀₋₁₎ alkyl group optionally substituted with a C₆₋₂₀ aryl group, a C₃₋₆ cycloalkyl group, a fluorine atom or a chlorine atom.

Yet another preferred starting material is characterized by the following formula (IIIc): 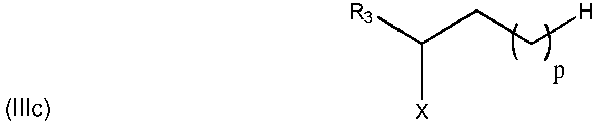 wherein p, X and R₃ are as defined above.

Further preferred starting material is characterized by the following formula (IIId): 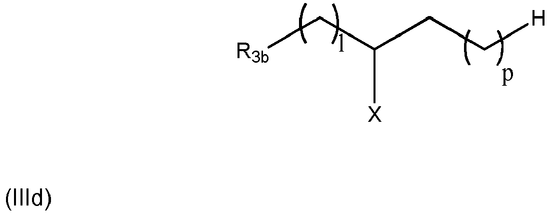 Wherein
p represents an integer of 0 to 3;
I represents an integer of 0 to 3;
p + I is less than or equal to 3;
X is as defined in claim 1;
R_{3b} represents a radical selected from a linear or branched C₁₋₍₂₀₋ₗ₎ alkyl group optionally substituted with a C₆₋₂₀ aryl group optionally substituted with a C₁₋₆alkylcarbonyloxy, a C₃₋₆ cycloalkyl group, a radical of formula -OC(O)R₅, a radical of formula -C(O)OR₅, R₅ being selected from the group consisting of methyl, and phenyl optionally substituted with one or more radicals selected from the group consisting of: fluoro, and C₁-₆alkyloxy, a fluorine atom or a chlorine atom.

Another preferred starting material is that wherein p is an integer from 0 to 8.

Another preferred starting material is that wherein p is an integer from 0 to 4 and each R₄ represents a hydrogen atom.

Another preferred starting material is that wherein X is bromine and the combination of solid and dotted lines represents a single bond. More preferred starting materials are characterized by this structural element in combination with any one of the other structural elements described herein as being preferred for the starting material (with the exception of the structural element directly underneath).

Another preferred starting material is that wherein X is hydrogen and the combination of solid and dotted lines represents a double bond. More preferred starting materials are characterized by this structural element in combination with any one of the other structural elements described herein as being preferred for the starting material (with the exception of the structural element directly above).

Another preferred starting material is that wherein p is an integer from 0 to 4 and each R₄ represents a hydrogen atom; and wherein R₃ is a C₁₋₁₀ alkyl group optionally substituted with a phenyl ring optionally substituted with a C₁₋₆alkylcarbonyloxy, a radical of formula -OC(O)R₅, or a radical of formula -C(O)OR₅, R₅ being selected from the group consisting of C₁₋₂₀alkyl, and phenyl optionally substituted with one or more radicals selected from the group consisting of: fluoro, and C₁-₆alkyloxy.

Another preferred starting material is that wherein p is an integer from 0 to 4 and each R₄ represents a hydrogen atom; R₃ is a C₁₋₁₀ alkyl group optionally substituted with a phenyl ring optionally substituted with a C₁₋₆alkylcarbonyloxy, a radical of formula -OC(O)R₅, or a radical of formula -C(O)OR₅, R₅ being selected from the group consisting of C₁-₂₀alkyl, and phenyl optionally substituted with one or more radicals selected from the group consisting of: fluoro, and C₁-₆alkyloxy; and X is bromine and the combination of solid and dotted lines represents a single bond; or, alternatively, X is hydrogen and the the combination of solid and dotted lines represents a double bond.

It is a characteristic of the method of the present invention that the direct carboxylation predominantly takes place at a terminal carbon atom of the starting material (as long as the starting material does not contain any electron withdrawing groups that are connected with the activating group only via secondary and/or tertiary carbon atoms but without quaternary carbons and/or as long as the reaction is carried out under kinetic control reaction conditions). This is independent of the position of the activating group (i.e. leaving group X or double bond) in the starting compound. For instance, in case of bromo-substituted heptanes as starting material, the method of the invention always yields octanoic acid with the carboxyl group in position 1 as the major product, irrespective whether the starting material is 2-bromoheptane, 3-bromoheptane or 4-bromoheptane.

The method of the present invention is regioconvergent such that a variety of different types of compounds are suitable as starting materials. Thus, it is possible to use a single compound as a starting material or a mixture of two or more compounds suitable as the starting material, while the same product is obtained.

Any mixture of starting materials can be used, provided that the individual members of the mixture are all suitable starting materials for the method of the present invention, as defined above. However, to accomplish the benefit of a regioconvergent reaction, mixtures of starting materials should be used, wherein the starting materials differ from each other only with respect to the activating group and/or position of activating group and only to such an extent that each starting material still yields the same reaction product. For instance, no benefits of a regioconvergent reaction can be accomplished if a mixture of starting materials is used, wherein starting materials differ from each other in size and/or substitution pattern.

Provided a suitable mixture of starting materials is used and due to the above-mentioned regioconvergent character of the method of the present invention, a possibly tedious and cumbersome purification of a mixture of compounds can hence be avoided which renders the method suitable for the preparation of carboxylic acids from mixtures of starting materials resulting from industrial processes, for instance.

A mixture of compounds of formula (III) where X is bromine and having the same aliphatic carbon skeleton can be obtained by radical bromination of an aliphatic compound.

### (B) The catalyst

The Nickel compound suitable as a catalyst promoting the method of the present invention is preferably prepared in situ by combining in a suitable solvent
(a) a nickel(II) salt,
(b) a reducing agent, and
(c) a ligand, and, optionally
(d) a hydrogen source
under the conditions specified below.

The following materials can advantageously be used.

### (a) Nickel(II) salt

The nickel(II) salt is selected from the group consisting of nickel (II) halide, nickel (II) acetylacetonate, nickel (II) sulphate, nickel (II) perchlorate, nickel (II) trifluoromethanesulfonate, nickel (II) hexafluoroacetylactonate, nickel (II) sulfamate, nickel (II) carbonate, nickel (II) oxalate, a compound of formula Ni(OOCR)₂ wherein R is a linear or branched C₁-₂₀alkyl or a C₃₋₈ cycloalkyl optionally substituted with one or more linear or branched C₁-₆alkyl chains, nickel hexafluorosilicate and solvates thereof. A preferred member of the group formed by the compounds of formula Ni(OOCR)₂ is nickel (II) acetate. It is also possible to use a mixture of two or more of these nickel(II) salts.

If the starting material is a bromoalkane or related compound with a leaving group X other than hydrogen, the nickel(II) salt is selected from nickel(II) bromide, nickel(II) chloride, nickel(II) iodide, nickel(II) acetylacetonate, nickel(II) perchlorate hexahydrate, nickel(II) triflate and solvates of such salts. Prefered solvates are nickel(II) chloride glyme solvate, nickel(II) bromide glyme solvate, nickel(II) bromide diglyme solvate. The use of nickel(II) iodide is particularly preferred. "Glyme" refers to 1,2-dimethoxyethane and "diglyme" refers to bis(2-methoxyethyl) ether.

If the starting material is an alkene, the same nickel(II) salts as listed above can be used. The use of nickel(II) iodide, nickel(II) bromide glyme solvate and nickel(II) bromide trihydrate is preferred.

### (b) Reducing agent

The reducing agent is used to convert Ni(II) to a Ni(0) species. Hence, it does not become part of the catalyst but it is nevertheless essential for forming or regenerating the catalyst. The reducing agent is selected from Mn, Zn, and mixtures thereof. The use of Mn is preferred. Alternatively, electrochemical reduction means may also be used, as described for instance in Beilstein J. Org. Chem. 2014, 10, 2484, Appl. Organometal. Chem. 2001, 15, 135 and Synlett 1990, 2, 361.

### (c) Ligand

The ligand is selected from the compounds of formula (I) 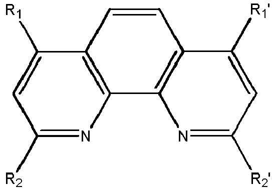 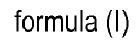 wherein
R₂ and R₂' are each independently selected from the group consisting of: hydrogen, a linear or branched C₁₋₁₂ alkyl group, a linear or branched C₁₋₁₂ haloalkyl group, a C₆₋₂₀ aryl group and a C₆₋₂₀ aryl group having at least one substituent selected from linear or branched C₁₋₆ alkyl groups, linear or branched C₁₋₆ alkoxy groups, linear or branched C₁₋₆ haloalkyl groups, fluoro, chloro and bromo; provided that R₂ and R₂'are not simultaneously hydrogen. Preferably, R₂ and R₂' are each independently selected from the group consisting of: a linear or branched C₁₋₁₂ alkyl group, a linear or branched C₁₋₁₂ haloalkyl group and a C₆₋₂₀ aryl group.

More preferably, R₂ and R₂' are each independently selected from the group consisting of linear or branched C₁₋₆ alkyl groups.

R₁ and R₁'are each independently selected from the group consisting of hydrogen, halogen, a linear or branched C₁₋₁₂ alkyl group, a linear or branched C₁₋₁₂ haloalkyl group, a C₆₋₂₀ aryl group and a C₆₋₂₀ aryl group having at least one substituent selected from linear or branched C₁₋₆ alkyl groups, linear or branched C₁₋₆ alkoxy groups, linear or branched C₁₋₆ haloalkyl groups, fluoro, chloro and bromo. Preferably, R₁ and R₁'are each independently selected from the group consisting of a C₆₋₂₀ aryl group and a C₆₋₂₀ aryl group having at least one substituent selected from linear or branched C₁₋₆ alkyl groups, linear or branched C₁₋₆ alkoxy groups, linear or branched C₁₋₆ haloalkyl groups, fluoro, chloro and bromo.

If the starting material is a bromoalkane or related compound with a leaving group X other than hydrogen, the following substitution patterns are advantageously used:
According to a first preferred embodiment, R₂ and R₂' are each independently selected from the group consisting of linear or branched C₁₋₆ alkyl groups, while R₁ and R₁' are each independently selected from the group consisting of hydrogen, phenyl, phenyl having at least one substituent selected from linear or branched C₁₋₆ haloalkyl groups and naphthyl.
According to a second preferred embodiment, R₂ and R₂' are selected from the group consisting of CH₃, C₂H₅, n-C₄H₉, n-C₆Hₗ₃, i-C₃H₇ and i-C₄H₉ while R₁ and R₁' both represent an aryl group and more preferably a phenyl group.
According to a third preferred embodiment, R₂ and R₂' represent both a methyl group and R₁ and R₁' are both a naphthyl group and more preferably R₁ and R₁' are both selected from the group consisting of 1-naphthyl and 2-naphthyl.

Among the above preferred meanings and embodiments, it is even more preferred if R₁ and R₁' are the same and/or if R₂ and R₂' are the same.

Particularly preferred are the ligands wherein R₂ and R₂' are the same and are selected from the group consisting of C₂H₅, n-C₄H₉, n-C₆H₁₃ and i-C₃H₇ while R₁ and R₁' both represent a phenyl group.

If the starting material is an alkene, the same substitution patterns as specified above can be used. In addition, the following substitution patterns are preferred:
According to a fourth preferred embodiment, R₂ and R₂' represent both a methyl group, ethyl group or propyl group and R₁ and R₁' are both selected from hydrogen, naphthyl and phenyl, the phenyl being optionally substituted with a radical selected from linear or branched C₁₋₆ alkyl groups, linear or branched C₁₋₆ alkoxy groups, linear or branched C₁₋₆ haloalkyl groups and halogen atoms, more preferably R₂ and R₂' represent both a methyl group and R₁ and R₁' are both selected from hydrogen, phenyl, C₁₋₆ alkoxy-substituted phenyl, C₁₋₆ haloalkyl-substituted phenyl and naphthyl group and even more preferably R₁ and R₁' are both selected from the group consisting of phenyl and methoxy-substituted phenyl, in particular phenyl, para-methoxy-substituted phenyl and ortho,para-dimethoxy-substituted phenyl.
According to a fifth preferred embodiment, at least one of R₂ and R₂' represents an n-butyl group while the other one of R₂ and R₂' may represent an n-butyl group or a methyl group, and wherein R₁ and R₁' both represent a phenyl group.

### (d) Hydrogen Source

Optionally a hydrogen source is also present, preferably when X is hydrogen and the combination of solid and dotted lines represents a double bond. The hydrogen source, if present, is selected from water and a compound of formula R₇R₈CBr-CHR₉R₁₀, wherein R₁₀, R₇, R₈, and R₉ are each independently selected from the group consisting of hydrogen, a linear or branched C₁₋₂₀ alkyl group optionally substituted with a radical selected from hydroxyl and C₁₋₂₀ alkyloxy, a C₃₋₈ cycloalkyl optionally substituted with a radical selected from hydroxyl and C₁-₂₀ alkyloxy, and a C₆₋₂₀aryl; or, alternatively, one or more of the pairs of R₈ and R₇, R₁₀ and R₉, R₇ and R₉ and R₈ and R₁₀, together with the carbon atoms to which they are attached form a ring system comprising from 1 to 3 rings, each ring may be isolated, or two or three rings may be fused or form a bridged structure, and comprising from 5 to 6 members selected from the group consisting of C, CH, and CH₂, each ring being independently unsaturated, saturated or aromatic; and each ring being further optionally substituted with one or more radicals selected from the group consisting of: hydroxyl, C₁-₆alkyl, and C₁₋₆alkyloxy.

Preferably, the hydrogen source is a compound selected from the compounds of formula R₇R₈CH-CH₂Br, wherein R₇ and R₈ are as defined above.

Generally, the hydrogen source can be used in an amount of 0-12 molar equivalents in relation to the amount of starting material being 1 molar equivalent.

If a bromoalkane or related compound with a leaving group X other than hydrogen is used as a starting material, water is tolerated in amounts of up to 8 molar equivalents in relation to the starting material being 1 molar equivalent.

If the starting material is an alkene, the hydrogen source is preferably selected from the group consisting of ethyl bromide, cyclohexyl bromide, dicyclohexylbromomethane, 2-bromocyclopentanol, 2-bromocyclohexanol and water. Among these hydrogen sources, the use of cyclohexyl bromide, water and 2-bromocyclopentanol is particularly preferred.

If the starting material is an alkene, it is preferred to use the hydrogen source in an amount of 0.9 to 11 molar equivalents relative to the starting material being 1 molar equivalent. Particularly preferable is the use of the hydrogen source in a molar amount of 1.8 to 5.5 equivalents relative to the starting material (alkene) being 1 molar equivalent.

It is also possible to use a combination of two or more of the above hydrogen sources.

### (e) Amounts and ratio of Ni(II) salt and compound of formula (I)

The Ni(II) salt is typically used in an amount of more than 0 mol% and 20 mol% or less with respect to the starting material being 100 mol%. It is advantageously used in an amount of 0.9 mol% to 11 mol% with respect to the starting material being 100 mol%.

The compound of formula (I) is used in an amount of more than 0 mol% and 80 mol% or less with respect to the starting material being 100 mol%. It is advantageously used in an amount of 0.9 mol% to 55 mol% with respect to the starting material being 100 mol%. It is advantageously used in an amount of 2 mol% to 30 mol% with respect to the starting material being 100 mol%.

The Ni(II) salt and the compound of formula (I) are advantageously used such that the molar ratio of compound of formula (I) : Ni(II) salt is in the range of from 1 to 5 and preferably in the range of from 1 to 3, more preferably in the range of from 1.5 to 2.

### (f) Amount of reducing agent

The reducing agent is advantageously used in an amount of 100 mol% to 550 mol% with respect to the starting material being 100 mol%.

If the starting material is an alkene, it is preferred that the reducing agent is used in an amount of 180 mol% to 550 mol% with respect to the starting material being 100 mol%.

### (g) Solvent

The nickel catalyst is preferably prepared in situ using the same solvent as specified below for the method of the invention. If the nickel catalyst is prepared in advance, it is also conceivable to rely on a different solvent. In such a case, it is nevertheless preferred to select the solvent among the different solvents described below for the method of the invention.

The in situ preparation of catalytically active Nickel species is described by Martin et al. in J. Am. Chem. Soc. 2015, 137, pages 6476-6479, for instance.

### (C) Reaction Temperature

The reaction temperature is not particularly restricted. It is advantageous to carry out the reaction at a temperature in the range of from -10 to 90°C.

If a bromoalkane or related compound with a leaving group X other than hydrogen is used as the starting material, it is preferred to employ a reaction temperature of from 0 to 90°C. If an alkene is used as the starting material, it is preferred to employ a reaction temperature of from 45 to 90°C.

The reaction temperature is preferably chosen in accordance with the starting material and the desired product, in the following manner:

| **Starting material** | **Desired product** | **Preferred Temperature [°C]** |
|---|---|---|
| Formula (III') or (III'"), compound has no electron withdrawing group | Formula (IIa), (IIb) or (IIc) | 0-90 |
| Formula (III') or (III'"), compound has electron withdrawing group | Formula (IIa), (IIb) or (IIc) | 0-20 |
| Formula (III') or (III'"), compound has electron withdrawing group | Formula (IId) or (IIe) | 35-90 |
| Formula (III"), compound has no electron withdrawing group | Formula (IIa), (IIb) or (IIc) | 25-90 |
| Formula (III") , compound has electron withdrawing group | Formula (IIa), (IIb) or (IIc) | 5-25 |
| Formula (III") , compound has electron withdrawing group | Formula (IId) or (IIe) | 40-90 |

### (D) Carbon Dioxide

Carbon dioxide is provided as a reagent. It is advantageous to provide carbon dioxide in gaseous form. The carbon dioxide pressure is not particularly limited. Supplying carbon dioxide at atmospheric pressure (1 atm) is advantageous as this pressure is easy to handle.

### (E) Solvent

The method of the present invention is carried out in the presence of a polar aprotic solvent. Said polar aprotic solvent can also be a mixture of two or more polar aprotic solvents.

One or more additional solvents selected from non-polar solvents and polar protic solvents may optionally be used together with the polar aprotic solvent mentioned above, provided the amount of such additional solvents is so small that it does not interfere with the method of the present invention. Water is a polar protic solvent. Nevertheless, to avoid confusion when determining relative amounts of components, water is to be regarded solely as a hydrogen source and not as a solvent in the context of the present invention.

The use of a solvent selected from DMF (N,N-Dimethylformamide), N,N-diethylformamide, DMSO (dimethylsulfoxide), DMA (N,N-dimethylacetamide), and NMP (N-methylpyrrolidine) is preferred but N,N-dibutylformamide and 1-Formylpyrrolidine can also be used.

It is also possible and preferred to use DMF in combination with another solvent selected from toluene, hexane and 1,4-dioxane. If such a mixture of DMF with another solvent is used, DMF and the other solvent should be present in relative volume ratio of from 1:0 to 1:1.2.

Yet further solvents may also be added in minor amounts (e.g. relative amounts of less than 10 wt.% based on the entire solvent amount) as long as they do not interfere with the reaction of the method of the present invention.

The use of DMF alone or of DMSO alone is particularly preferred. This applies especially to those embodiments, wherein the starting material is a bromoalkane or related compound with a leaving group X other than hydrogen.

### (F) Products

The method of the invention predominantly favors the formation of terminal carboxylic acids, in particular when the compound of formula (III) does not comprise an electron withdrawing group.

Consequently, when the conditions relative to the compound of formula (III) for the predominant formation of a compound of formula (IIa) described above are fulfilled, the method of the invention provides a compound of formula (IIa) as the predominant product.

Preferably, u=0, so that the above formula (IIa) can be simplified to formula (IIa'):

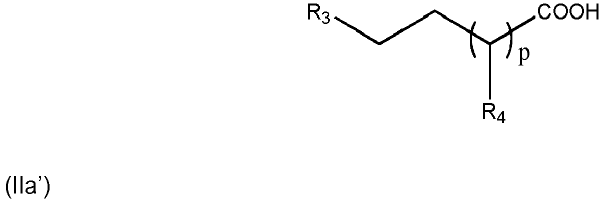

Preferably, a starting material is used wherein the R₄ radical of the terminal carbon atom represents hydrogen, so that the reaction provides a compound of formula (IIa"). In a preferred variant, u = 0, so that the product is of formula (IIa"'):

R₃-CH₂₋ᵤ(R₃')ᵤ-CH₂₋ᵤ(R₄)ᵤ-(CHR₄)ₚ₋₁-CH₂-COOH (IIa")

R₃-CH₂-CH₂-(CHR₄)ₚ₋₁-CH₂-COOH (IIa"')

wherein R₃, R₃', p, u and R₄ are as described in any of the embodiments of the invention described above for the compound of formula (III).

In a similar manner, when the conditions relative to the compound of formula (III) for the predominant formation of a compound of formula (IIb) described above are fulfilled, and in particular, when Y is not a C₃₋₈ cycloalkyl, the method of the invention is preferably carried out with a starting material such that it provides a compound of formula (IIb) as described above where the COOH group is attached to the remainder of the molecule through a methylene diradical.

A preferred reaction product of formula (IIb) is a product wherein u=0, so that the formula (IIb) can be simplified to (IIb'): 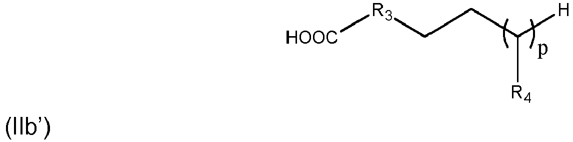 wherein R₃, p and R₄ are as described above for the compound of formula (IIb).

A preferred reaction product of formula (IIc) is a product wherein u=0, so that the formula (IIc) can be simplified to (IIc'): 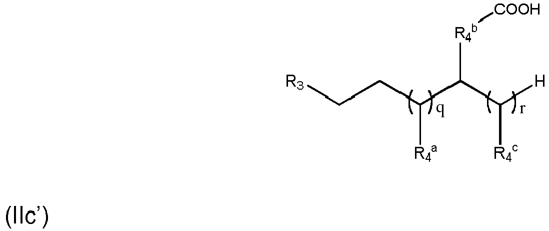 wherein R₃, q, r, R₄ₐ, R_{4b} and R_{4c} are as described above for the compound of formula (IIc).

In a similar manner, when the conditions relative to the compound of formula (III) for the predominant formation of a compound of formula (IIc) described above are fulfilled, the method of the invention is preferably carried out with a starting material such that it provides a compound of formula (IIc') and more preferably a compound of formula (IIc"), for which u = 0.

R₃-CH₂₋ᵤ(R₃')ᵤ-CH₂₋ᵤ(R₄)ᵤ-(CHR₄ₐ)_{q}-CH(R_{4b}'(CH₂-COOH))-(CHR_{4c})ᵣ-H (IIc")

R₃-CH₂-CH₂-(CHR₄ₐ)_{q}-CH(R_{4b}'(CH₂-COOH))-(CHR_{4c})ᵣ-H (IIc"')

wherein R₃, R₃', R₄ₐ, R_{4c}, q and r are as described in any of the embodiments of the invention described above for the compound of formula (IIc) and R_{4b}' is either a single bond or a linear or branched C₁₋₁₉ alkyl.

A preferred reaction product of formula (IId) is a product wherein u=0, so that the formula (IId) can be simplified to (IId'): 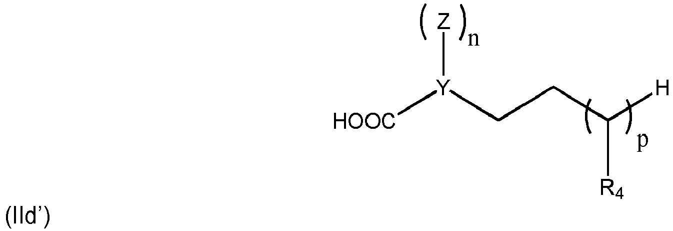 wherein Y, Z, R₄, n and p are as described above for the compound of formula (IId).

### Particularly preferred Embodiments

It is particularly preferred to carry out the method of the present invention in such a manner that two or more of the features of the invention are selected among the preferred embodiments.

For instance, it is particularly preferred to use the starting materials that are specified above as being preferred (or more preferred or the like) together with a nickel catalyst that is prepared in situ using a preferred Ni(II) salt and/or a preferred ligand of formula (I) and/or the preferred reducing agent Mn and/or a preferred hydrogen source and/or the use of these components in the preferred relative amounts.

Similarly, it is particularly preferred to carry out the preferred embodiments of the method of the invention (in terms of starting material and/or in situ prepared nickel catalyst) in a reaction solvent that is specified above as being preferred and/or at a reaction temperature that is specified as being preferred.

Of course, the degree of preference increases with an increasing number of features selected from the features described above as being preferred.

Thus, for instance, it is particularly preferred to carry out the method of the present invention using a secondary bromoalkane or alkene of formula (III) as a starting material, more preferably a secondary bromoalkane or alkene of formula (III) which does not contain an electron withdrawing group, and to form the nickel catalyst in situ and carry out the direct carboxylation reaction;
using a nickel(II) salt selected from nickel(II) bromide, nickel(II) chloride, nickel(II) iodide, nickel(II) acetylacetonate, nickel(II) perchlorate hexahydrate, nickel(II) triflate, nickel(II) chloride glyme solvate, nickel(II) bromide glyme solvate, and nickel(II) bromide diglyme solvate, in an amount of 0.9 mol% to 11 mol% with respect to the starting material being 100 mol%; and
using Mn as the reducing agent in an amount of 90 mol% to 550 mol% with respect to the starting material being 100 mol%; and
using a ligand of formula (I), wherein the ligand fulfills one or more of the following conditions (i) to (ix):
(i) R₂ and R₂' are each independently selected from the group consisting of: hydrogen, a linear or branched C₁₋₁₂ alkyl group, a linear or branched C₁₋₁₂ haloalkyl group and a C₆₋₂₀ aryl group;
(ii) R₂ and R₂' are each independently selected from the group consisting of linear or branched C₁₋₆ alkyl groups;
(iii) R₁ and R₁'are each independently selected from the group consisting of a C₆₋₂₀ aryl group and a C₆₋₂₀ aryl group having at least one substituent selected from linear or branched C₁₋₆ alkyl groups, linear or branched C₁₋₆ alkoxy groups, linear or branched C₁₋₆ haloalkyl groups, fluoro, chloro and bromo;
(iv) R₂ and R₂' are each independently selected from the group consisting of linear or branched C₁₋₆ alkyl groups, while R₁ and R₁' are each independently selected from the group consisting of hydrogen, phenyl, phenyl having at least one substituent selected from linear or branched C₁₋₆ haloalkyl groups and naphthyl;
(v) R₂ and R₂' are selected from the group consisting of CH₃, C₂H₅, n-C₄H₉, n-C₆Hₗ₃, i-C₃H₇ and i-C₄H₉ while R₁ and R₁' both represent an aryl group and more preferably a phenyl group;
(vi) R₂ and R₂' represent both a methyl group and R₁ and R₁' are both a naphthyl group and more preferably R₁ and R₁' are both selected from the group consisting of 1-naphthyl and 2-naphthyl;
(vii) R₂ and R₂' are the same and are selected from the group consisting of C₂H₅, n-C₄H₉, n-C₆H₁₃ and i-C₃H₇ while R₁ and R₁' both represent a phenyl group; or
(viii) R₂ and R₂' represent both a methyl group and R₁ and R₁' are both selected from hydrogen, phenyl, C₁₋₆ alkoxy-substituted phenyl, C₁₋₆ haloalkyl-substituted phenyl and naphthyl group and more preferably R₁ and R₁' are both selected from the group consisting of phenyl and methoxy-substituted phenyl, in particular phenyl, para-methoxy-substituted phenyl and ortho,para-dimethoxy-substituted phenyl; or
(ix) at least one of R₂ and R₂' represents an n-butyl group while the other one of R₂ and R₂' may represent an n-butyl group or a methyl group, and wherein R₁ and R₁' both represent a phenyl group;
in an amount of 0.9 mol% to 55 mol% with respect to the starting material being 100 mol%; wherein preferably R₁ and R₁' are the same and/or R₂ and R₂' are the same; and
optionally using a hydrogen source, which is selected from water and the group of compounds characterized by the general formula R₇R₈CH-CH₂-Br, wherein R₇ and R₈ are independently selected from hydrogen, a linear or branched C₁₋₂₀ alkyl group optionally substituted with a radical selected from hydroxyl and C₁₋₂₀ alkyloxy, a C₃₋₈ cycloalkyl optionally substituted with a radical selected from hydroxyl and C₁₋₂₀ alkyloxy, and a C₆₋₂₀ aryl; and
carrying out the reaction at a temperature in the range of from 0 to 90°C; and
carrying out the reaction in a solvent consisting of or containing a solvent selected from DMF (N,N-Dimethylformamide), N,N-diethylformamide, DMSO (dimethylsulfoxide), DMA (N,N-dimethylacetamide), NMP (N-methylpyrrolidine), N,N-dibutylformamide and 1-formylpyrrolidine.

Another particularly preferred embodiment is characterized by a feature combination relating to the use of a bromoalkane of formula (III), more preferably a secondary bromoalkane of formula (III) which does not contain an electron withdrawing group, such as especially a bromoalkane of formula (IIIc) or (IIId) which does not contain an electron withdrawing group as specified above or below, as a starting material and to form the nickel catalyst in situ and carry out the direct carboxylation reaction
using a nickel(II) salt selected from nickel(II) bromide, nickel(II) chloride, nickel(II) iodide, nickel(II) acetylacetonate, nickel(II) perchlorate hexahydrate, nickel(II) triflate, nickel(II) chloride glyme solvate, nickel(II) bromide glyme solvate, and nickel(II) bromide diglyme solvate, in an amount of 0.9 mol% to 11 mol% with respect to the starting material being 100 mol%; and
using Mn as the reducing agent in an amount of 100 mol% to 550 mol% with respect to the starting material being 100 mol%; and
using a ligand of formula (I), wherein the ligand fulfills one or more of the following conditions:
(a) R₂ and R₂' are each independently selected from the group consisting of linear or branched C₁₋₆ alkyl groups, while R₁ and R₁' are each independently selected from the group consisting of hydrogen, phenyl, phenyl having at least one substituent selected from linear or branched C₁₋₆ haloalkyl groups and naphthyl,
(b) R₂ and R₂' are selected from the group consisting of CH₃, C₂H₅, n-C₄H₉, n-C₆Hₗ₃, i-C₃H₇ and i-C₄H₉ while R₁ and R₁' both represent an aryl group and more preferably a phenyl group,
(c) R₂ and R₂' represent both a methyl group and R₁ and R₁' are both a naphthyl group and more preferably R₁ and R₁' are both selected from the group consisting of 1-naphthyl and 2-naphthyl,
(d) R₂ and R₂' are the same and are selected from the group consisting of C₂H₅, n-C₄H₉, n-C₆H₁₃ and i-C₃H₇ while R₁ and R₁' both represent a phenyl group,
in an amount of 0.9 mol% to 55 mol% with respect to the starting material being 100 mol%; wherein preferably R₁ and R₁' are the same and/or R₂ and R₂' are the same;
carrying out the reaction at a temperature in the range of from 14 to 66°C; and
carrying out the reaction in a solvent consisting of or containing a solvent selected from DMF (N,N-Dimethylformamide), N,N-diethylformamide, DMSO (dimethylsulfoxide), DMA (N,N-dimethylacetamide), NMP (N-methylpyrrolidine), N,N-dibutylformamide and 1-formylpyrrolidine.

Yet another particularly preferred embodiment is characterized by a feature combination relating to the use of an alkene of formula (III), wherein X represents hydrogen and the bond with the dashed line represents a carbon-carbon double bond, such as especially an alkene of formula (IIIa) or (IIIb) as specified above or below, which preferably does not contain an electron withdrawing group, as a starting material and to form the nickel catalyst in situ and carry out the direct carboxylation reaction
using a nickel(II) salt selected from nickel(II) bromide, nickel(II) chloride, nickel(II) iodide, nickel(II) acetylacetonate, nickel(II) perchlorate hexahydrate, nickel(II) triflate, nickel(II) chloride glyme solvate, nickel(II) bromide glyme solvate, and nickel(II) bromide diglyme solvate, in an amount of 0.9 mol% to 11 mol% with respect to the starting material being 100 mol%; and
using Mn as the reducing agent in an amount of 180 mol% to 550 mol% with respect to the starting material being 100 mol%; and
using a ligand of formula (I), wherein the ligand fulfills one or more of the following conditions:
(1) R₂ and R₂' represent both a methyl group and R₁ and R₁' are both selected from hydrogen, phenyl, C₁₋₆ alkoxy-substituted phenyl, C₁₋₆ haloalkyl-substituted phenyl and naphthyl group and more preferably R₁ and R₁' are both selected from the group consisting of phenyl and methoxy-substituted phenyl, in particular phenyl, para-methoxy-substituted phenyl and ortho,para-dimethoxy-substituted phenyl,
(2) at least one of R₂ and R₂' represents an n-butyl group while the other one of R₂ and R₂' may represent an n-butyl group or a methyl group, and wherein R₁ and R₁' both represent a phenyl group,
in an amount of 0.9 mol% to 55 mol% with respect to the starting material being 100 mol%; wherein preferably R₁ and R₁' are the same and/or R₂ and R₂' are the same;
carrying out the reaction at a temperature in the range of from 45 to 90°C;
optionally in the presence of a hydrogen source that is preferably selected from the group consisting of ethyl bromide, cyclohexyl bromide, dicyclohexylbromomethane, 2-bromocyclopentanol, 2-bromocyclohexanol and water; and
carrying out the reaction in a solvent consisting of or containing a solvent selected from DMF (N,N-Dimethylformamide), N,N-diethylformamide, DMSO (dimethylsulfoxide), DMA (N,N-dimethylacetamide), NMP (N-methylpyrrolidine).

Another particularly preferred embodiment of the present invention involves using as the starting material a starting material of formula (III') or (III'"), wherein the group R₃ contains at least one electron withdrawing group Z, and forming the nickel catalyst in situ and carrying out the direct carboxylation reaction
using a nickel(II) salt selected from nickel(II) bromide, nickel(II) chloride, nickel(II) iodide, nickel(II) acetylacetonate, nickel(II) perchlorate hexahydrate, nickel(II) triflate, nickel(II) chloride glyme solvate, nickel(II) bromide glyme solvate, and nickel(II) bromide diglyme solvate, in an amount of 0.9 mol% to 11 mol% with respect to the starting material being 100 mol%; and
using Mn as the reducing agent in an amount of 100 mol% to 550 mol% with respect to the starting material being 100 mol%; and
using a ligand of formula (I), wherein the ligand fulfills one or more of the following conditions:
(a) R₂ and R₂' are each independently selected from the group consisting of linear or branched C₁₋₆ alkyl groups, while R₁ and R₁' are each independently selected from the group consisting of hydrogen, phenyl, phenyl having at least one substituent selected from linear or branched C₁₋₆ haloalkyl groups and naphthyl,
(b) R₂ and R₂' are selected from the group consisting of CH₃, C₂H₅, n-C₄H₉, n-C₆Hₗ₃, i-C₃H₇ and i-C₄H₉ while R₁ and R₁' both represent an aryl group and more preferably a phenyl group,
(c) R₂ and R₂' represent both a methyl group and R₁ and R₁' are both a naphthyl group and more preferably R₁ and R₁' are both selected from the group consisting of 1-naphthyl and 2-naphthyl,
(d) R₂ and R₂' are the same and are selected from the group consisting of C₂H₅, n-C₄H₉, n-C₆H₁₃ and i-C₃H₇ while R₁ and R₁' both represent a phenyl group,
in an amount of 0.9 mol% to 55 mol% with respect to the starting material being 100 mol%; wherein preferably R₁ and R₁' are the same and/or R₂ and R₂' are the same;
carrying out the reaction at a temperature in the range of from -10 to 25°C if a product of formula (IIa), (IIb) or (IIc) is desired, or, alternatively, carrying out the reaction at a temperature in the range of from 35 to 90°C if a product of formula (IId) or (IIe) is desired; and
carrying out the reaction in a solvent consisting of or containing a solvent selected from DMF (N,N-Dimethylformamide), N,N-diethylformamide, DMSO (dimethylsulfoxide), DMA (N,N-dimethylacetamide), NMP (N-methylpyrrolidine), N,N-dibutylformamide and 1-formylpyrrolidine.

According to a further particularly preferred embodiment, the method involves using as a starting material a compound of formula (III"), wherein the group R₃ contains at least one electron withdrawing group Z, and to form the nickel catalyst in situ and carry out the direct carboxylation reaction
using a nickel(II) salt selected from nickel(II) bromide, nickel(II) chloride, nickel(II) iodide, nickel(II) acetylacetonate, nickel(II) perchlorate hexahydrate, nickel(II) triflate, nickel(II) chloride glyme solvate, nickel(II) bromide glyme solvate, and nickel(II) bromide diglyme solvate, in an amount of 0.9 mol% to 11 mol% with respect to the starting material being 100 mol%; and
using Mn as the reducing agent in an amount of 180 mol% to 550 mol% with respect to the starting material being 100 mol%; and
using a ligand of formula (I), wherein the ligand fulfills one or more of the following conditions:
(1) R₂ and R₂' represent both a methyl group and R₁ and R₁' are both selected from hydrogen, phenyl, C₁₋₆ alkoxy-substituted phenyl, C₁₋₆ haloalkyl-substituted phenyl and naphthyl group and more preferably R₁ and R₁' are both selected from the group consisting of phenyl and methoxy-substituted phenyl, in particular phenyl, para-methoxy-substituted phenyl and ortho,para-dimethoxy-substituted phenyl,
(2) at least one of R₂ and R₂' represents an n-butyl group while the other one of R₂ and R₂' may represent an n-butyl group or a methyl group, and wherein R₁ and R₁' both represent a phenyl group,
in an amount of 0.9 mol% to 55 mol% with respect to the starting material being 100 mol%; wherein preferably R₁ and R₁' are the same and/or R₂ and R₂' are the same;
carrying out the reaction at a temperature in the range of from -5 to 25°C if a product of formula (IIa), (IIb) or (IIc) is desired, or, alternatively, carrying out the reaction at a temperature in the range of from 40 to 90°C if a product of formula (IId) or (IIe) is desired;
optionally in the presence of a hydrogen source that is preferably selected from the group consisting of ethyl bromide, cyclohexyl bromide, dicyclohexylbromomethane, 2-bromocyclopentanol, 2-bromocyclohexanol and water; and
carrying out the reaction in a solvent consisting of or containing a solvent selected from DMF (N,N-Dimethylformamide), N,N-diethylformamide, DMSO (dimethylsulfoxide), DMA (N,N-dimethylacetamide), NMP (N-methylpyrrolidine).

### Examples

### Example 1: Carboxylation of 2-bromoheptane

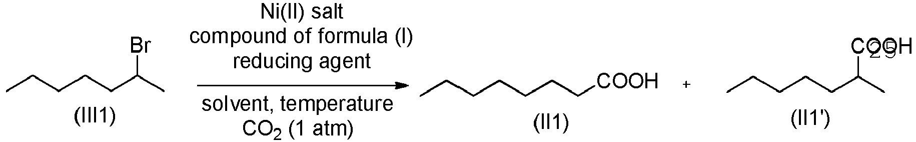

### General procedure

An oven-dried schlenk tube containing a stirring bar was charged with the nickel (II) salt indicated in Tables 1-7 in the amount indicated in Tables 1-7, the compound of formula (I) as indicated in Tables 1-7 in the amount indicated in Tables 1-7 and the reducing agent indicated in Tables 1-7 in the amount indicated in Tables 1-7. The schlenk tube was then evacuated and back-filled under a carbon dioxide flow (this sequence was repeated three times) and finally an atmospheric pressure of CO₂ was established. A 1 M solution of the bromoheptane compound (III1) in the solvent indicated in Tables 1-7 was subsequently added by syringe and the solution was stirred for 17 h at the temperature indicated in Tables 1-7. The mixture was then carefully quenched with 2 M aqueous hydrochloric solution to hydrolyze the resulting carboxylate and extracted with ethyl acetate. A sample of such obtained solution was next analyzed by gas chromatography using anisole as an internal standard. The resulting carboxylic acid can further be purified by silica gel column chromatography using Hexanes/Ethyl Acetate (3/1) as elution system.

### Table 1: Scope of nickel (II) salt precursor

In Entries 1-10:
(i) the compound of formula (I) is that wherein R₁ and R₁' are both phenyl, and R₂ and R₂' are both methyl, and its amount is one fourth the amount of 2-bromoheptane engaged in the reaction;
(ii) the amount of nickel (II) salt is one tenth the amount of 2-bromoheptane engaged in the reaction;
(iii) the reducing agent is manganese and its amount is 3.6 times the amount of 2-bromoheptane engaged in the reaction;
(iv) the solvent is N,N-dimethylformamide; and
(v) the temperature is 50 °C.

| **Entry** | **Nickel (II) salt** | **Yield of (II1) (%)** | **Ratio (II1):(II1')** |
|---|---|---|---|
| 1 | NiCl₂ · glyme | 38 | 19:1 |
| 2 | NiBr₂ · glyme | 45 | 12:1 |
| 3 | NiBr₂ · diglyme | 46 | 13:1 |
| 4 | Nil₂ | 49 | 25:1 |
| 5 | NiBr₂ | 45 | 15:1 |
| 6 | NiCl₂ | 15 | >15:1 |
| 7 | NiF₂ | 7 | >7:1 |
| 8 | Ni(acac)₂ (acac=acetylacetonate) | 29 | 15:1 |
| 9 | Ni(ClO0₄)₂ · 6 H₂O | 16 | 15:1 |
| 10 | Ni(OTf)₂ | 34 | 16:1 |

| | | | |
|---|---|---|---|
| OTf refers to triflate, i.e. -O-S(O)₂-CF₃ | | | |

### Table 2: Scope of solvent

In Entries 11-17:
(i) the compound of formula (I) is that wherein R₁ and R₁' are both phenyl, and R₂ and R₂' are both methyl, and its amount is one fourth the amount of 2-bromoheptane engaged in the reaction;
(ii) the nickel (II) salt is nickel (II) iodide and the amount of nickel (II) iodide is one tenth the amount of 2-bromoheptane engaged in the reaction;
(iii) the reducing agent is manganese and its amount is 3.6 times the amount of 2-bromoheptane engaged in the reaction;
(v) the temperature is 50 °C.

| **Entry** | **Solvent** | **Yield of (II1) (%)** | **Ratio (II1):(II1')** |
|---|---|---|---|
| 11 | DMF | 49 | 25:1 |
| 12 | DMF:Toluene (1:1) | 20 | 19:1 |
| 13 | DMF:Hexane (1:1) | 32 | 11:1 |
| 14 | DMF:1,4-Dioxane (1:1) | 16 | >16:1 |
| 15 | DMSO | 41 | 40:1 |
| 16 | DMA | 39 | 38:1 |
| 17 | NMP | 34 | 16:1 |

| | | | |
|---|---|---|---|
| DMF is N,N-dimethylformamide, DMSO is dimethylsulfoxide, DMA is N,N-dimethylacetamide and NMP is N-methylpyrrolidine. | | | |

### Table 3: Scope of temperature

In Entries 19-22:
(i) the compound of formula (I) is that wherein R₁ and R₁' are both phenyl, and R₂ and R₂' are both methyl, and its amount is one fourth the amount of 2-bromoheptane engaged in the reaction;
(ii) the nickel (II) salt is nickel (II) iodide and the amount of nickel (II) iodide is one tenth the amount of 2-bromoheptane engaged in the reaction;
(iii) the reducing agent is manganese and its amount is 3.6 times the amount of 2-bromoheptane engaged in the reaction;
(v) the solvent is DMF.

| **Entry** | **Temperature (°C)** | **Yield of (II1) (%)** | **Ratio (II1):(II1')** |
|---|---|---|---|
| 18 | 30 | 9 | >9:1 |
| 19 | 40 | 47 | 46:1 |
| 20 | 50 | 49 | 25:1 |
| 21 | 60 | 40 | 20:1 |
| 22 | 70 | 29 | 28:1 |

### Table 4: Water/moisture tolerance

In Entries 23-26:
(i) the compound of formula (I) is that wherein R₁ and R₁' are both phenyl, and R₂ and R₂' are both methyl, and its amount is one fourth the amount of 2-bromoheptane engaged in the reaction;
(ii) the nickel (II) salt is nickel (II) iodide and the amount of nickel (II) iodide is one tenth the amount of 2-bromoheptane engaged in the reaction;
(iii) the reducing agent is manganese and its amount is 3.6 times the amount of 2-bromoheptane engaged in the reaction;
(v) the solvent is DMF;
(vi) the temperature is 50 °C; and
(vii)the amount of water indicated in Table 4 has been added to the reaction mixture.

| **Entry** | **Added water (with respect to 2-bromoheptane)** | **Yield of (II1) (%)** | **Ratio (II1):(II1')** |
|---|---|---|---|
| 23 | 2 equivalents | 19 | >19:1 |
| 24 | 4 equivalents | 14 | >14:1 |
| 25 | 6 equivalents | 20 | >20:1 |
| 26 | 8 equivalents | 17 | >17:1 |

### Table 5: Scope of compound of formula (I)

In Entries 27-39:
(i) the compound of formula (I) is that wherein R₁, R₁', R₂ and R₂' are as indicated in Table 5, and its amount is one fourth the amount of 2-bromoheptane engaged in the reaction;
(ii) the nickel (II) salt is nickel (II) iodide and the amount of nickel (II) iodide is one tenth the amount of 2-bromoheptane engaged in the reaction;
(iii) the reducing agent is manganese and its amount is 3.6 times the amount of 2-bromoheptane engaged in the reaction;
(v) the solvent is DMF;
(vi) the temperature is 30 °C.

| **Entry** | **Compound of formula (I)** | | | | **Yield of (II1) (%)** | **Ratio (II1):(II1')** |
|---|---|---|---|---|---|---|
| | **R₁** | **R₁'** | **R₂** | **R₂'** | | |
| 27 | H | H | H | H | 0 | - |
| 28 | H | H | CH₃ | CH₃ | 4 | >10:1 |
| 29 | C₆H₅ | C₆H₅ | CH₃ | CH₃ | 7 | >10:1 |
| 30 | *p*-CF₃-C₆H₄ | *p*-CF₃-C₆H₄ | CH₃ | CH₃ | 8 | >10:1 |
| 31 | 1-naphthyl | 1-naphthyl | CH₃ | CH₃ | 26 | 21:1 |
| 32 | 2-naphthyl | 2-naphthyl | CH₃ | CH₃ | 29 | 24:1 |
| 33 | C₆H₅ | C₆H₅ | C₂H₅ | C₂H₅ | 69 | 30:1 |
| 34 | C₆H₅ | C₆H₅ | *n*-C₄H₉ | *n*-C₄H₉ | 65 | 29:1 |
| 35 | C₆H₅ | C₆H₅ | *n*-C₄H₉ | H | 8 | >10:1 |
| 36 | C₆H₅ | C₆H₅ | *n*-C₆H₁₃ | *n*-C₆H₁₃ | 72 | 28:1 |
| 37 | C₆H₅ | C₆H₅ | *i*-C₃H₇ | *i*-C₃H₇ | 62 | 21:1 |
| 38 | C₆H₅ | C₆H₅ | *i*-C₄H₉ | *i*-C₄H₉ | 29 | 29:1 |
| 39 | Cl | Cl | CH₃ | CH₃ | 8 | >8:1 |

### Table 6: Screening of the ratio of nickel (II) salt to compound of formula (I)

In Entries 40-49:
(i) the compound of formula (I) is that wherein R₁ and R₁' are phenyl and R₂ and R₂' are n-hexyl (entry 36), and its amount is as indicated in Table 6;
(ii) the nickel (II) salt is nickel (II) iodide and its amount is as indicated in Table 6;
(iii) the reducing agent is manganese and its amount is 3.6 times the amount of 2-bromoheptane engaged in the reaction;
(v) the solvent is DMF;
(vi) the temperature is as indicated in Table 6.

| **Entry** | **Temp. (°C)** | **Amount of Nil₂ (mol %)** | **Amt. cpd. formula (I) (mol %)** | **Ratio Ni:(I)** | **Yield of (II1) (%)** | **Ratio (II1):(II1')** |
|---|---|---|---|---|---|---|
| 40 | 50 | 10 | 15 | 0.67 | 48 | 24:1 |
| 41 | 27 | 5 | 5 | 1.00 | 10 | >10:1 |
| 42 | 27 | 5 | 6.25 | 0.80 | 55 | 26:1 |
| 43 | 27 | 5 | 7.5 | 0.67 | 35 | 34:1 |
| 44 | 27 | 5 | 8.75 | 0.57 | 56 | 55:1 |
| 45 | 25 | 10 | 17.5 | 0.57 | 78 | 39:1 |
| 46 | 25 | 10 | 15 | 0.67 | 92 | 50:1 |
| 47 | 25 | 5 | 8.75 | 0.57 | 85 | 43:1 |
| 48 | 25 | 2.5 | 4.40 | 0.57 | 96 | 40:1 |
| 49 | 25 | 1 | 1.8 | 0.56 | 40 | 39:1 |

### Table 7: Screening of loading of reducing agent

In Entries 50-55:
(i) the compound of formula (I) is that wherein R₁ and R₁' are phenyl and R₂ and R₂' are n-hexyl (entry 36), and its amount is 4.40 mol%;
(ii) the nickel (II) salt is nickel (II) iodide and its amount is 2.5 mol%;
(iii) the reducing agent is as indicated in Table 7 and its amount is as indicated in Table 7;
(v) the solvent is DMF;
(vi) the temperature is 25 °C.

| **Entry** | **Reducing agent** | **Amount of reducing agents (in equivalents)** | **Yield of (II1) (%)** | **Ratio (II1):(II1')** |
|---|---|---|---|---|
| 50 | Mn | 1 | 55 | 30:1 |
| 51 | Mn | 1.5 | 75 | 35:1 |
| 52 | Mn | 2 | 88 | 45:1 |
| 53 | Mn | 3 | 94 | 40:1 |
| 54 | Mn | 3.5 | 96 | 40:1 |
| 55^{a} | Zn | 3.5 | 9 | >9:1 |

| | | | | |
|---|---|---|---|---|
| ^{a}In entry 55, 10 mol% of Nil₂ and 15 mol% of compound of formula (I) were used. | | | | |

### Example 2: Carboxylation of 3-bromoheptane

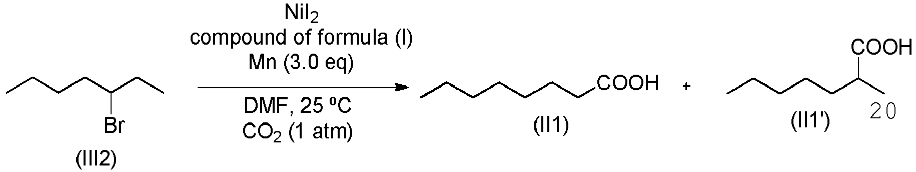

3-bromoheptane (IIIb) was carboxylated to 1-octanoic acid following the general procedure of Example 1, using:
(i) a compound of formula (I) wherein R₁ and R₁' are phenyl and R₂ and R₂' are n-hexyl, in an amount of 8.80 mol%;
(ii) 5.0 mol% of Nil₂ as nickel (II) salt;
(iii) 3.0 equivalents of manganese as reducing agent
(iv) DMF as a solvent and at a temperature of 25 °C.

In these conditions the process of the invention provides octanoic acid in 81% isolated yield, with a ratio of (II1):(II1') of 25 to 1.

### Example 3: Carboxylation of 4-bromoheptane

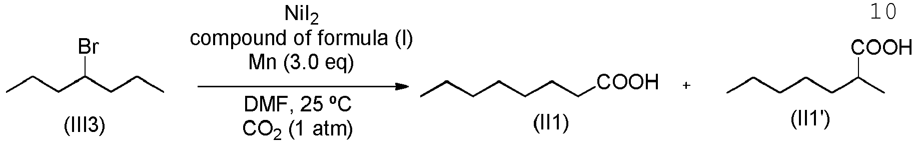

4-bromoheptane (IIIc) was carboxylated to 1-octanoic acid following the general procedure of Example 1, using:
(i) a compound of formula (I) wherein R₁ and R₁' are phenyl and R₂ and R₂' are n-hexyl, in an amount of 8.80 mol%;
(ii) 5.0 mol% of Nil₂ as nickel (II) salt;
(iii) 3.0 equivalents of manganese as reducing agent
(iv) DMF as a solvent and at a temperature of 25 °C.

In these conditions the process of the invention provides octanoic acid in 72% isolated yield, with a ratio of (II1):(II1') of 24 to 1.

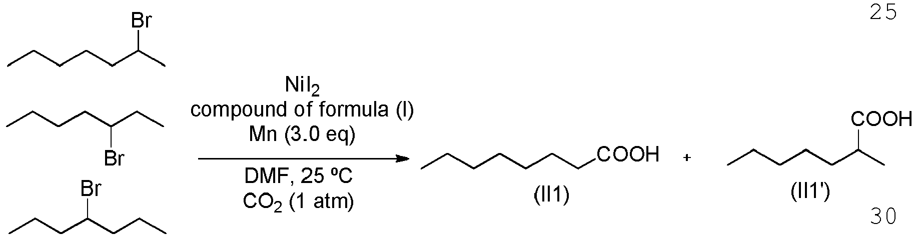

### Example 4: Carboxylation of a mixture of bromoheptane isomers

An equimolar mixture (1:1:1) of 2-bromoheptane, 3-bromoheptane and 4-bromoheptane was carboxylated to 1-octanoic acid following the general procedure of Example 1, using:
(i) a compound of formula (I) wherein R₁ and R₁' are phenyl and R₂ and R₂' are n-hexyl, in an amount of 8.80 mol%;
(ii) 5.0 mol% of Nil₂ as nickel (II) salt;
(iii) 3.0 equivalents of manganese as reducing agent
(iv) DMF as a solvent and at a temperature of 25 °C.

In these conditions the process of the invention provides octanoic acid in 85% isolated yield, with a ratio of (II1):(II1') of 25 to 1.

### Example 5: Carboxylation of methyl 5-bromohexanoate

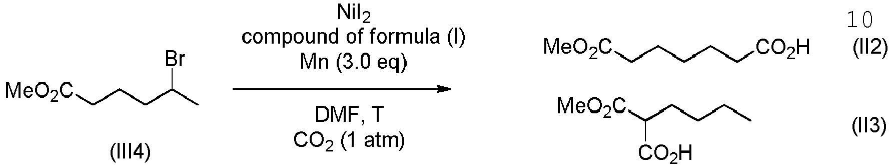

### Table 8: Scope of temperature

In Entries 56-61:
(i) the compound of formula (I) is that wherein R₁ and R₁' are phenyl and R₂ and R₂' are n-hexyl (entry 36), and its amount is 4.40 mol%;
(ii) the nickel (II) salt is nickel (II) iodide and its amount is 2.5 mol%;
(iii) the reducing agent is manganese and its amount is 3.0 times the amount of methyl 5-bromohexanoate engaged in the reaction;
(iv) the solvent is DMF;
(vi) the temperature is as indicated in Table 8.

| **Entry** | **Temperature (°C)** | **Yield of (II2)+(II3) (%)** | **Ratio (II2):(II3)** |
|---|---|---|---|
| 56 | 10 | 86 | 85:15 |
| 57 | 15 | 90 | 75:25 |
| 58 | 25 | 96 | 59:41 |
| 59 | 31 | 94 | 50:50 |
| 60 | 38 | 85 | 14:86 |
| 61 | 42 | 81 | 4:96 |

### Example 6: Carboxylation of various alkyl bromides

### Table 9: Substrate scope

In Entries 62-63, the carboxylation method was carried out using the compound of formula (III) described in Table 9 and following the general procedure of Example 5, entry 56.

In Entries 64-65, the carboxylation method was carried out using the compound of formula (III) described in Table 9 and following the general procedure of Example 2, but at a temperature of 10 °C.

| Entry | Compound (III) | Products (II) | Yield | Ratio l/b |
|---|---|---|---|---|
| 62 | 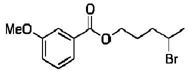 | 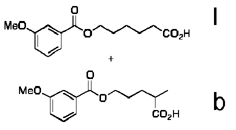 | 74 | >19:1 |
| 63 | 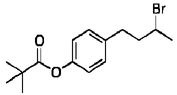 | 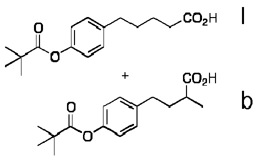 | 61 | >19:1 |
| 64 | 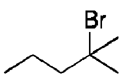 | 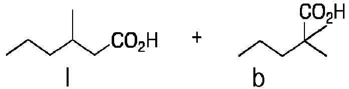 | 30 | >19:1 |
| 65 | 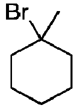 | 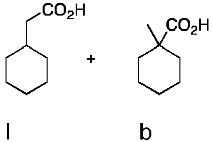 | 50 | >19:1 |

### Example 7: Carboxylation of 1-octene

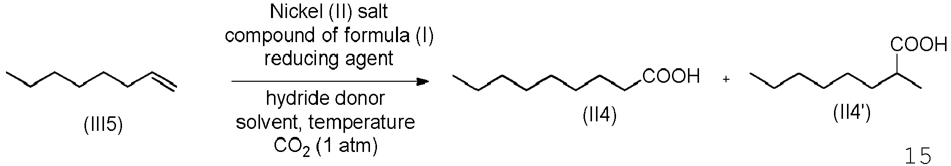

### General procedure

An oven-dried schlenk tube containing a stirring bar was charged with the nickel (II) salt indicated in Tables 10-15 in the amount indicated in Tables 10-15, the compound of formula (I) as indicated in Tables 10-15 in the amount indicated in Tables 10-15 and the reducing agent indicated in Tables 8-14 in the amount indicated in Tables 10-15. The schlenk tube was then evacuated and back-filled under a carbon dioxide flow (this sequence was repeated three times) and finally an atmospheric pressure of CO₂ was established. A 1 M solution of the alkene compound in the solvent indicated in Tables 10-15 and further comprising the hydrogen source indicated in Tables 10-15 in the amount indicated in Tables 9-14 was subsequently added by syringe and the solution was stirred for 40 h at the temperature indicated in Tables 10-15. The mixture was then carefully quenched with 2 M aqueous hydrochloric solution to hydrolyze the resulting carboxylate and extracted with ethyl acetate. A sample of such obtained solution was next analyzed by gas chromatography using anisole as an internal standard. The resulting carboxylic acid can further be purified by silica gel column chromatography using Hexanes/Ethyl Acetate (3/1) as elution system.

### Table 10: Scope of the hydrogen source

In Entries 1-13:
(i) the compound of formula (I) is that wherein R₁ and R₁' are both phenyl, and R₂ and R₂' are both methyl, and its amount is one fourth the amount of 1-octene engaged in the reaction;
(ii) the nickel (II) salt is nickel (II) iodide and its amount is one tenth the amount of 1-octene engaged in the reaction;
(iii) the reducing agent is manganese and its amount is 3.6 times the amount of 1-octene engaged in the reaction;
(iv) the solvent is N,N-dimethylformamide; and
(v) the temperature is 50 °C.

| **Entry** | **Hydrogen source / amount of hydrogen source (equivalents)** | **Yield of (II4) (%)** | **Ratio (II4):(II4')** |
|---|---|---|---|
| 1 | Ethyl bromide / 1 | 23 | >23:1 |
| 2 | Cyclohexyl bromide / 2 | 59 | >59:1 |
| 3 | Cyclohexyl bromide / 5 | 84 | 42:1 |
| 4 | Dicyclohexylbromomethane / 2 | 22 | >22:1 |
| 5 | 2-bromocyclopentanol / 2 | 41 | 40:1 |
| 6 | 2-bromocyclohexanol / 2 | 39 | 38:1 |
| 7 | Water / 0.5 | 5 | >5:1 |
| 8 | Water / 1 | 4 | >4:1 |
| 9 | Water / 2 | 14 | >14:1 |
| 10 | Water / 4 | 17 | >17:1 |
| 11 | Water / 6 | 31 | 30:1 |
| 12 | Water/8 | 23 | 22:1 |
| 13 | Water / 10 | 19 | >19:1 |

### Table 11: Scope of nickel (II) salts

In Entries 14-17:
(i) the compound of formula (I) is that wherein R₁ and R₁' are both phenyl, and R₂ and R₂' are both methyl, and its amount is one fourth the amount of 1-octene engaged in the reaction;
(ii) the amount of nickel (II) salt is one tenth the amount of 1-octene engaged in the reaction;
(iii) the reducing agent is manganese and its amount is 3.6 times the amount of 1-octene engaged in the reaction;
(iv) the solvent is N,N-dimethylformamide;
(v) the temperature is 50 °C;
(vi) the hydrogen source is water, in an amount of 6 equivalents.

| **Entry** | **Nickel (II) salt** | **Yield of (II4) (%)** | **Ratio (II4):(II4')** |
|---|---|---|---|
| 14 | Nil₂ | 31 | 30:1 |
| 15 | NiBr₂ | 5 | >5:1 |
| 16 | NiBr₂ · glyme | 13 | >13:1 |
| 17 | NiBr₂ · 3H₂O | 16 | >16:1 |

### Table 12: Scope of temperature

In Entries 18-21:
(i) the compound of formula (I) is that wherein R₁ and R₁' are both phenyl, and R₂ and R₂' are both methyl, and its amount is one fourth the amount of 1-octene engaged in the reaction;
(ii) the nickel (II) salt is nickel (II) iodide and its amount is one tenth the amount of 1-octene engaged in the reaction;
(iii) the reducing agent is manganese and its amount is 3.6 times the amount of 1-octene engaged in the reaction;
(iv) the solvent is N,N-dimethylformamide;
(v) the hydrogen source is water, in an amount of 6 equivalents.

| **Entry** | **Temperature (°C)** | **Yield of (II4) (%)** | **Ratio (II4):(II4')** |
|---|---|---|---|
| 18 | 50 | 31 | 30:1 |
| 19 | 60 | 25 | 24:1 |
| 20 | 70 | 32 | 31:1 |
| 21 | 80 | 25 | 24:1 |

### Table 13: Screening of solvents

In Entries 22-27:
(i) the compound of formula (I) is that wherein R₁ and R₁' are both phenyl, and R₂ and R₂' are both methyl, and its amount is one fourth the amount of 1-octene engaged in the reaction;
(ii) the nickel (II) salt is nickel (II) iodide and its amount is one tenth the amount of 1-octene engaged in the reaction;
(iii) the reducing agent is manganese and its amount is 3.6 times the amount of 1-octene engaged in the reaction;
(iv) the temerpature is 50 °C;
(v) the hydrogen source is water, in an amount of 6 equivalents.

| **Entry** | **Solvent** | **Yield of (II4) (%)** | **Ratio (II4):(II4')** |
|---|---|---|---|
| 22 | DMF | 31 | 30:1 |
| 23 | N,N-diethylformamide | 18 | >18:1 |
| 24 | N,N-dibutylformamide | 5 | >5:1 |
| 25 | 1-Formylpyrrolidine | 5 | >5:1 |
| 26 | N,N-dimethylacetamide | 20 | >20:1 |
| 27 | N-methylpyrrolidine | 19 | >19:1 |

### Table 14: Scope of loading of reducing agent

In Entries 28-32:
(i) the compound of formula (I) is that wherein R₁ and R₁' are both phenyl, and R₂ and R₂' are both methyl, and its amount is one fourth the amount of 1-octene engaged in the reaction;
(ii) the nickel (II) salt is nickel (II) iodide and its amount is one tenth the amount of 1-octene engaged in the reaction;
(iii) the reducing agent is manganese;
(iv) the solvent is N,N-dimethylformamide;
(v) the temperature is 50 °C;
(vi) the hydrogen source is water, in an amount of 6 equivalents.

| **Entry** | **Loading of reducing agent (equivalents)** | **Yield of (II4) (%)** | **Ratio (II4):(II4')** |
|---|---|---|---|
| 28 | 1 | 9 | >9:1 |
| 29 | 2 | 34 | 33:1 |
| 30 | 3 | 26 | 25:1 |
| 31 | 3.6 | 31 | 30:1 |
| 32 | 5 | 35 | 34:1 |

### Table 15: Scope of loading of compound of formula (I)

In Entries 33-37:
(i) the compound of formula (I) is that wherein R₁ and R₁' are both phenyl, and R₂ and R₂' are both methyl;
(ii) the nickel (II) salt is nickel (II) iodide and its amount is one tenth the amount of 1-octene engaged in the reaction (10 mol%);
(iii) the reducing agent is manganese and its amount is 3.6 equivalents;
(iv) the solvent is N,N-dimethylformamide;
(v) the temperature is 50 °C;
(vi) the hydrogen source is water, in an amount of 6 equivalents.

| **Entry** | **Loading of compound of formula (I) (mol%)** | **Yield of (II4) (%)** | **Ratio (II4):(II4')** |
|---|---|---|---|
| 33 | 15 | 16 | >16:1 |
| 34 | 20 | 25 | 24:1 |
| 35 | 25 | 31 | 30:1 |
| 36 | 35 | 51 | 50:1 |
| 37 | 50 | 27 | 26:1 |

### Table 16: Scope of compound of formula (I)

In Entries 38-47:
(i) the amount of the compound of formula (I) is 35 mol%;
(ii) the nickel (II) salt is nickel (II) iodide and its amount is one tenth the amount of 1-octene engaged in the reaction (10 mol%);
(iii) the reducing agent is manganese and its amount is 3.6 equivalents;
(iv) the solvent is N,N-dimethylformamide;
(v) the temperature is 50 °C;
(vi) the hydrogen source is water, in an amount of 6 equivalents.

| **Entry** | **Compound of formula (I)** | | | | **Yield of (II4) (%)** | **Ratio (II4):(II4')** |
|---|---|---|---|---|---|---|
| | **R₁** | **R₁'** | **R₂** | **R₂'** | | |
| 38 | H | H | CH₃ | CH₃ | 18 | >50:1 |
| 39 | C₆H₅ | C₆H₅ | CH₃ | CH₃ | 51 | 50:1 |
| 40 | *p*-CF₃-C₆H₄ | *p*-CF₃-C₆H₄ | CH₃ | CH₃ | 22 | >50:1 |
| 41 | 1-naphthyl | 1-naphthyl | CH₃ | CH₃ | 17 | >50:1 |
| 42 | 2-naphthyl | 2-naphthyl | CH₃ | CH₃ | 26 | >50:1 |
| 43 | C₆H₅ | C₆H₅ | *n*-C₄H₉ | *n*-C₄H₉ | 28 | >50:1 |
| 44 | C₆H₅ | C₆H₅ | *n*-C₄H₉ | CH₃ | 31 | 30:1 |
| 45^{a} | C₆H₅ | C₆H₅ | CH₃ | CH₃ | 27 | 26:1 |
| 46^{a} | *p*-OCH₃-C₆H₄ | *p*-OCH₃-C₆H₄ | CH₃ | CH₃ | 49 | 48:1 |
| 47^{a} | 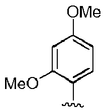 | 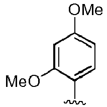 | CH₃ | CH₃ | 56 | 29:1 |

| | | | | | | |
|---|---|---|---|---|---|---|
| ^{a}15 h reaction time instead of 40 h. | | | | | | |

### Example 8: Carboxylation of 1-heptene

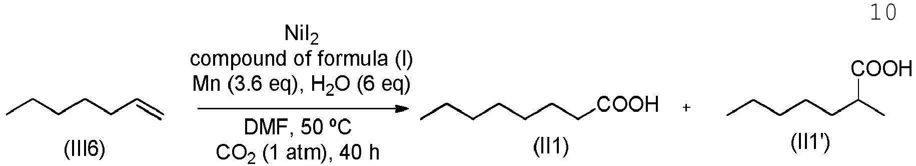

1-heptene (IIIe) was carboxylated to 1-octanoic acid following the general procedure of Example 5, using:
(i) a compound of formula (I) wherein R₁ and R₁' are phenyl and R₂ and R₂' are methyl, in an amount of 35 mol%;
(ii) 10 mol% of Nil₂ as nickel (II) salt;
(iii) 3.6 equivalents of manganese as reducing agent;
(iv) 6 equivalents of water as hydrogen source; and
(iv) DMF as a solvent and at a temperature of 50 °C.

In these conditions the process of the invention provides octanoic acid in 49% isolated yield, with a ratio of (II1):(II1') of 49 to 1.

### Example 9: Carboxylation of 3,3-dimethylbut-1-ene

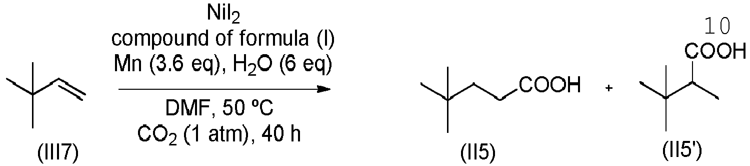

3,3-dimethylbut-1-ene (IIIf) was carboxylated to the corresponding carboxylic acid following the general procedure of Example 5, using:
(i) a compound of formula (I) wherein R₁ and R₁' are phenyl and R₂ and R₂' are methyl, in an amount of 35 mol%;
(ii) 10 mol% of Nil₂ as nickel (II) salt;
(iii) 3.6 equivalents of manganese as reducing agent;
(iv) 6 equivalents of water as hydrogen source; and
(iv) DMF as a solvent and at a temperature of 50 °C.

In these conditions the process of the invention provides compound (II5) in 30% isolated yield, with a ratio of (II5):(II5') of >30:1.

### Example 10: Carboxylation of 4-octene

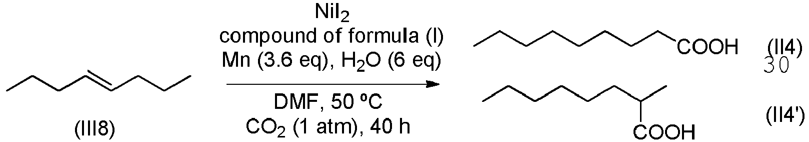

4-octene (IIIg) was carboxylated to the corresponding carboxylic acid following the general procedure of Example 5, using:
(i) a compound of formula (I) wherein R₁ and R₁' are phenyl and R₂ and R₂' are methyl, in an amount of 35 mol%;
(ii) 10 mol% of Nil₂ as nickel (II) salt;
(iii) 3.6 equivalents of manganese as reducing agent;
(iv) 6 equivalents of water as hydrogen source; and
(iv) DMF as a solvent and at a temperature of 50 °C.

In these conditions the process of the invention provides compound (II4) in 10% isolated yield, with a ratio of (II4):(II4') of >10:1.

### Example 11: Carboxylation of 2-heptene

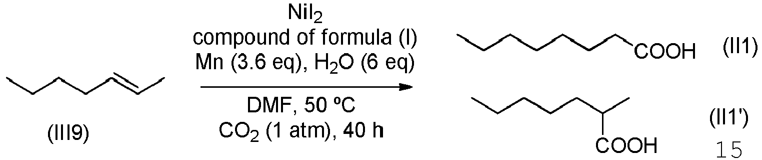

2-heptene (IIIh) was carboxylated to the corresponding carboxylic acid following the general procedure of Example 5, using:
(i) a compound of formula (I) wherein R₁ and R₁' are phenyl and R₂ and R₂' are methyl, in an amount of 35 mol%;
(ii) 10 mol% of Nil₂ as nickel (II) salt;
(iii) 3.6 equivalents of manganese as reducing agent;
(iv) 6 equivalents of water as hydrogen source; and
(iv) DMF as a solvent and at a temperature of 50 °C.

In these conditions the process of the invention provides compound (II1) in 15% isolated yield, with a ratio of (II1):(II1') of >15:1.

### Example 12: Carboxylation of but-3-en-1-ylbenzene

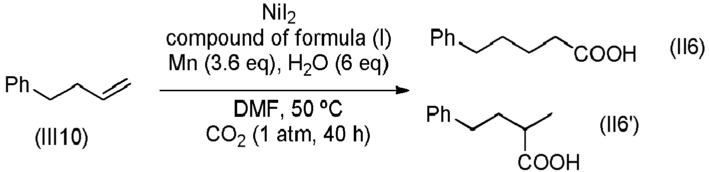

But-3-en-1-ylbenzene (IIIi) was carboxylated to the corresponding carboxylic acid following the general procedure of Example 5, using:
(i) a compound of formula (I) wherein R₁ and R₁' are phenyl and R₂ and R₂' are methyl, in an amount of 35 mol%;
(ii) 10 mol% of Nil₂ as nickel (II) salt;
(iii) 3.6 equivalents of manganese as reducing agent;
(iv) 6 equivalents of water as hydrogen source; and
(iv) DMF as a solvent and at a temperature of 50 °C.

In these conditions the process of the invention provides compound (II6) in 40% isolated yield, with a ratio of (II6):(II6') of 39 to 1.

## Claims

1. Method of direct carboxylation of a compound represented by formula (III) 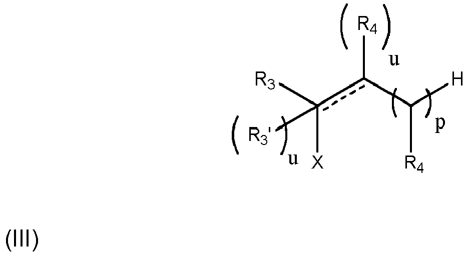 wherein
p is an integer from 0 to 15;
u is 0 or 1;
each R₄ represents a radical independently selected from the group consisting of
hydrogen,
a linear or branched C₁₋₂₀ alkyl group optionally carrying one or more substituents selected from fluoro and chloro,
a linear or branched C₁₋₂₀ alkyloxy group;
X represents hydrogen, a bromine atom, an iodine atom, a phenylsulfonate wherein the phenyl ring is optionally substituted with a methyl group, a methylsulfonate and a trifluoromethylsulfonate;
with the proviso that when X represents hydrogen, then the bond represented by the combination of a solid line and a dotted line is a double bond, and u is 0; and that when X is not hydrogen, then the bond represented by the combination of a solid line and a dotted line is a single bond, and u is 1;
R₃ and R₃' are each independently selected from hydrogen and a radical of formula -Y(-Z)ₙ,
wherein
Y is selected from a linear C₁₋₂₀ alkyl, a branched C₃₋₂₀ alkyl and a C₃₋₈ cycloalkyl;
n is an integer from 0 to the number of carbon atoms in Y;
each Z is independently selected from the group consisting of:
fluoro,
chloro,
a radical of formula -OR₅, a radical of formula -C(O)R₅, a radical of formula - OC(O)R₅, a radical of formula -C(O)OR₅, a radical of formula -NHC(O)R₅, a radical of formula -C(O)NHR₅, a radical of formula -NHC(O)OR₅, a radical of formula -OC(O)NHR₅, R₅ being selected from the group consisting of a linear or branched C₁₋₂₀ alkyl groups, and a cyclic group comprising 1 ring or 2 to 5 condensed rings,
wherein each ring has 5 or 6 members selected from the group consisting of C, CH, CH₂, O, S, N and NR₆,
wherein R₆ is selected from hydrogen and C₁₋₆alkyl,
each ring being independently unsaturated, saturated or aromatic;
and each ring being further optionally substituted with one or more radicals selected from the group consisting of: fluoro, chloro, C₁₋₆alkyl, C₁₋₆haloalkyl, C₁₋₆alkyloxy, C₁₋₆alkylcarbonyl, C₁₋₆alkylcarbonyloxy, C₁₋₆alkylcarbonylamino, and C₁₋₆alkylaminocarbonyl,
an aldehyde group,
nitro,
cyano,
a group of formula -SO₂R₁₁
wherein R₁₁ is selected from C₁₋₆alkyl, C₁₋₆haloalkyl, phenyl and phenyl substituted with one or more C₁₋₆alkyl groups; and
a cyclic group comprising 1 ring or 2 to 5 condensed rings,
wherein each ring has 5 or 6 members selected from the group consisting of C, CH, CH₂, O, S, N and NR₆,
wherein R₆ is selected from hydrogen and C₁₋₆alkyl,
each ring being independently unsaturated, saturated or aromatic;
and each ring being further optionally substituted with one or more radicals selected from the group consisting of: C₁₋₆alkyl, C₁₋₆haloalkyl, C₁₋₆alkyloxy, C₁₋₆alkylcarbonyl, C₁₋₆alkylcarbonyloxy, C₁₋₆alkylcarbonylamino, C₁₋₆alkylaminocarbonyl, -Rₐ-R_{b},
wherein Rₐ is a single bond or a diradical selected from -O-, -S-, - C(=O)-, -C(=O)-O-, -O-C(=O)-, -C(=O)-NH-, -NH-C(=O)-, -O-C(=O)-NH-, -NH-C(=O)-O-, -O-C(=O)-O-, -S(O)₂-, -S(O)₂-NH-, - NH-S(O)₂-, -N=N-, -NH-C(=O)-NH-,
and
wherein R_{b} is selected from a C₆₋₁₀ aryl group, a C₃₋₈ cycloalkyl group optionally containing one or two double bonds, or a saturated or unsaturated heterocyclic group containing 5 to 7 ring members wherein each ring member is selected from C, CH, CH₂, O, S, N and NR₆, wherein R₆ is selected from hydrogen and C₁₋₆alkyl;
or; alternatively, R₃ and R₃' together with the carbon atom to which they are attached form a cyclic group comprising 1 ring or 2 to 5 condensed rings, wherein each ring has 5 or 6 members selected from the group consisting of C, CH, CH₂, O, S, N and NR₆, wherein R₆ is selected from hydrogen and C₁₋₆alkyl, each ring being independently unsaturated, or saturated; and each ring being further optionally substituted with one or more radicals selected from the group consisting of: C₁₋₆alkyl, C₁₋₆haloalkyl, C₁₋₆alkyloxy, C₁₋₆alkylcarbonyl, C₁₋₆alkylcarbonyloxy, C₁₋₆alkylcarbonylamino, C₁₋₆alkylaminocarbonyl, -Rₐ-R_{b},
wherein Rₐ is a single bond or a diradical selected from -O-, -S-, - C(=O)-, -C(=O)-O-, -O-C(=O)-, -C(=O)-NH-, -NH-C(=O)-, -O-C(=O)-NH-, -NH-C(=O)-O-, -O-C(=O)-O-, -S(O)₂-, -S(O)₂-NH-, - NH-S(O)₂-, -N=N-, -NH-C(=O)-NH-,
and wherein R_{b} is selected from a C₆₋₁₀ aryl group, a C₃₋₈ cycloalkyl group optionally containing one or two double bonds, or a saturated or unsaturated heterocyclic group containing 5 to 7 ring members wherein each ring member is selected from C, CH, CH₂, O, S, N and NR₆, wherein R₆ is selected from hydrogen and C₁₋₆alkyl;
with the proviso that R₃ is not hydrogen if X is not hydrogen;
the method comprising the step of reacting the compound of formula (III) with carbon dioxide in the presence of a catalytically effective amount of a nickel catalyst and in the presence of a polar aprotic solvent,
wherein the nickel catalyst is obtainable by contacting in a polar aprotic solvent:
(a) a nickel(II) salt selected from the group consisting of nickel (II) halide, nickel (II) acetylacetonate, nickel (II) sulphate, nickel (II) perchlorate, nickel (II) trifluoromethanesulfonate, nickel (II) hexafluoroacetylacetonate, nickel (II) sulfamate, nickel (II) carbonate, nickel (II) oxalate, a compound of formula Ni(OOCR)₂ wherein R is a linear or branched C₁₋₂₀alkyl or a C₃₋₈ cycloalkyl optionally substituted with one or more linear or branched C₁₋₆alkyl chains, nickel hexafluorosilicate and solvates thereof;
(b) a reducing agent selected from Mn and Zn or an electrochemical reducing means, and
(c) a ligand of formula (I) 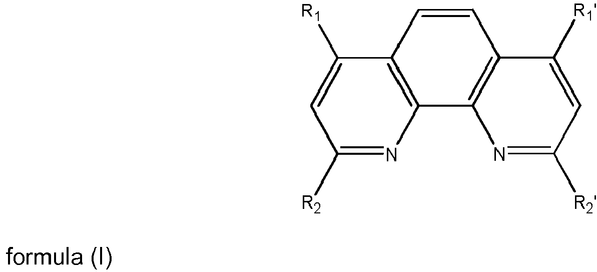 wherein
R₂ and R₂'are each independently selected from the group consisting of: hydrogen, a linear or branched C₁₋₁₂ alkyl group, a linear or branched C₁₋₁₂ haloalkyl group, a C₆₋₂₀ aryl group and a C₆₋₂₀ aryl group having at least one substituent selected from linear or branched C₁₋₆ alkyl groups, linear or branched C₁₋₆ alkoxy groups, linear or branched C₁₋₆ haloalkyl groups, fluorine, chlorine and bromine; provided that when R₂ or, respectively, R₂', is hydrogen, then R₂', or, respectively, R₂, is not hydrogen;
R₁ and R₁' are each independently selected from the groups consisting of hydrogen, halogen, a linear or branched C₁₋₁₂ alkyl group, a linear or branched C₁₋₁₂ haloalkyl group, a C₆₋₂₀ aryl group and a C₆₋₂₀ aryl group having at least one substituent selected from linear or branched C₁₋₆ alkyl groups, linear or branched C₁₋₆ alkoxy groups, linear or branched C₁₋₆ haloalkyl groups and halogen atoms; and
(d) optionally a hydrogen source selected from water, a compound of formula R₇R₈CH-CH₂Br, and a compound of formula R₇R₈CBr-CHR₉R₁₀, wherein R₁₀, R₇, R₈, and R₉ are each independently selected from the group consisting of hydrogen, a linear or branched C₁₋₂₀ alkyl optionally substituted with a radical selected from hydroxyl and C₁₋₂₀ alkyloxy, a C₃₋₈ cycloalkyl optionally substituted with a radical selected from hydroxyl and C₁₋₂₀ alkyloxy, and a C₆₋₂₀ aryl; or, alternatively,
one or more of the pairs of R₈ and R₇, R₁₀ and R₈, R₇ and R₉ and R₈ and R₁₀, together with the carbon atoms to which they are attached form a ring system comprising from 1 to 3 rings, each ring being isolated, fused or bridged, and comprising from 5 to 6 members selected from the group consisting of C, CH, and CH₂, each ring being independently unsaturated, saturated or aromatic; and each ring being further optionally substituted with one or more radicals selected from the group consisting of: hydroxyl, C₁₋₆alkyl, and C₁₋₆alkyloxy.

2. Method according to claim 1, wherein the direct carboxylation yields a reaction product selected from the following formulae (IIa'), (IIb'), (IIc') and (IId'): 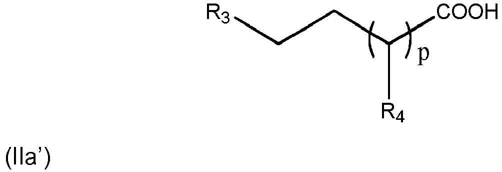 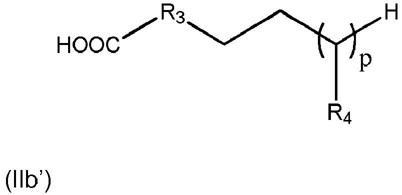 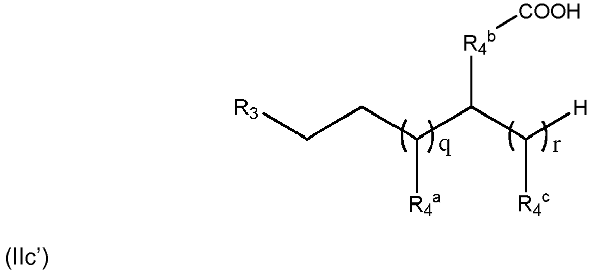 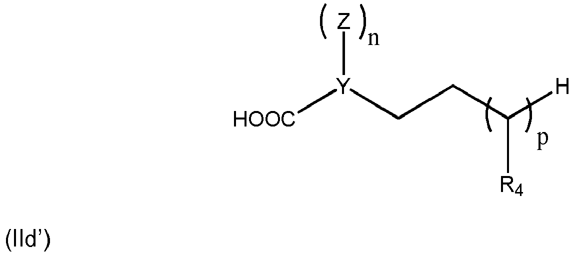 wherein R₃, R₄, Y, Z, p and n have the same meanings as specified in claim 1 and wherein each of R₄a, R₄b and R₄c has the same meaning as specified in claim 1 with respect to R₄ but with the proviso that R₄b in formula (IIc') is selected such that the carboxyl group is attached to a terminal aliphatic carbon atom of the R_{4b} group that is derived from a primary aliphatic carbon atom in the starting material, and that is connected with the position of the leaving group X or double bond in the starting material via a chain of secondary and tertiary carbon atoms that is not interrupted by any quaternary carbon atoms,
wherein the carboxyl group in the products of formulae (IIa', (IIb'), (IIc') and (IId') is preferably bonded via a methylene group -CH₂-.

3. Method as defined in claim 1 or claim 2, wherein the compound of formula (III) is represented by formula (IIIa), 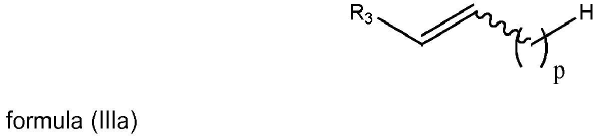 wherein the double bond is in cis or trans configuration and wherein R₃ and p are as specified in claim 1.

4. Method as defined in claim 3, wherein the compound of formula (III) is a compound of formula (IIIb) 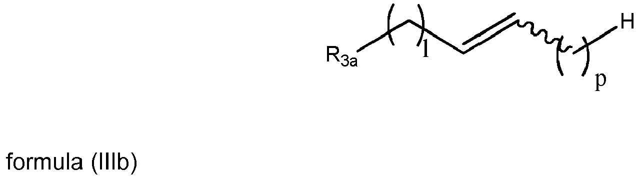 wherein
p represents an integer of 0 to 3;
l represents an integer of 0 to 3;
p + l is less than or equal to 3;
R₃ₐ represents a radical selected from a linear or branched C₁₋₍₂₀₋ₗ₎ alkyl group optionally substituted with a C₆₋₂₀ aryl group, a C₃₋₆ cycloalkyl group, a fluorine atom or a chlorine atom.

5. Method as defined in claims 1, 2, 3 or 4, wherein the compound of formula (III) is a mixture of two or more compounds of formula III), wherein in each of these compounds X represents hydrogen and the bond represented by the combination of a solid line and a dotted line is a double bond and wherein these compounds have the same total number of carbon atoms and where groups R₃ and R₄ are such that the two or more compounds of formula (III) differ from each other only with respect to the position of the double bond and such that the same reaction product is obtained for each of these compounds.

6. Method as defined in claim 1 or claim 2, wherein the compound of formula (III) is represented by formula (IIIc), 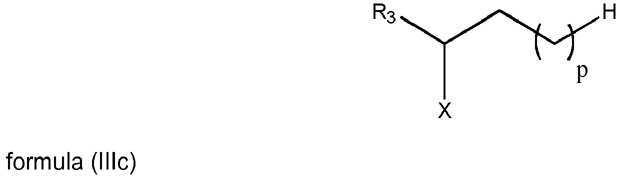 wherein p, X and R₃ are as defined in claim 1.

7. Method as defined in claim 6, wherein the compound of formula (III) is a compound of formula (IIId) 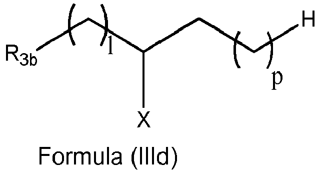 wherein
p represents an integer of 0 to 3;
l represents an integer of 0 to 3;
p + l is less than or equal to 3;
X is as defined in claim 1;
R_{3b} represents a radical selected from a linear or branched C₁₋(₂₀₋ₗ) alkyl group optionally substituted with a C₆₋₂₀ aryl group optionally substituted with a C₁₋₆alkylcarbonyloxy, a C₃₋₆ cycloalkyl group, a radical of formula -OC(O)R₅, a radical of formula -C(O)OR₅, R₅ being selected from the group consisting of methyl, and phenyl optionally substituted with one or more radicals selected from the group consisting of:
fluoro, and C₁₋₆alkyloxy, a fluorine atom or a chlorine atom.

8. Method as defined in claims 1, 2, 6 or 7, wherein the compound of formula (III) is a mixture of two or more compounds, which two or more compounds have a group X other than hydrogen as well as the same total number of carbon atoms and where groups R₃ and R₄ are such that the two or more compounds differ from each other only with respect to the position of the leaving group X and such that the same reaction product is obtained for each of these compounds.

9. Method as defined in any one of the preceding claims, wherein the reaction conditions are selected such that one or more of the following features is fulfilled:
(i) the catalyst is formed using Ni(II) iodide;
(ii) Mn is used as the reducing agent;
(iii) a ligand of formula (I) is used, wherein R₂ and R₂' are each independently selected from the group consisting of: hydrogen, a linear or branched C₁₋₁₂ alkyl group, a linear or branched C₁₋₁₂ haloalkyl group and a C₆₋₂₀ aryl group;
(iv) a ligand of formula (I) is used, wherein R₂ and R₂' are each independently selected from the group consisting of linear or branched C₁₋₆ alkyl groups; and
(v) a ligand of formula (I) is used, wherein R₁ and R₁' are each independently selected from the group consisting of a C₆₋₂₀ aryl group and a C₆₋₂₀ aryl group having at least one substituent selected from linear or branched C₁₋₆ alkyl groups, linear or branched C₁₋₆ alkoxy groups, linear or branched C₁₋₆ haloalkyl groups, fluoro, chloro and bromo.

10. Method as defined in any one of the preceding claims, wherein a ligand is used, which contains variable groups selected from the following alternative options:
(a) R₂ and R₂' are each independently selected from the group consisting of linear or branched C₁₋₆ alkyl groups, while R₁ and R₁' are each independently selected from the group consisting of hydrogen, phenyl, phenyl having at least one substituent selected from linear or branched C₁₋₆ haloalkyl groups and naphthyl;
(b) R₂ and R₂' are selected from the group consisting of CH₃, C₂H₅, n-C₄H₉, n-C₆H₁₃, i-C₃H₇ and *i*-C₄H₉ while R₁ and R₁' both represent an aryl group and more preferably a phenyl group;
(c) R₂ and R₂' represent both a methyl group and R₁ and R₁' are both a naphthyl group and more preferably R₁ and R₁' are both selected from the group consisting of 1-naphthyl and 2-naphthyl;
(d) R₂ and R₂' are the same and are selected from the group consisting of C₂H₅, n-C₄H₉, n-C₆H₁₃ and i-C₃H₇ while R₁ and R₁' both represent a phenyl group;
(e) R₂ and R₂' represent both a methyl group and R₁ and R₁' are both selected from hydrogen, phenyl, C₁₋₆ alkoxy-substituted phenyl, C₁₋₆ haloalkyl-substituted phenyl and naphthyl group and more preferably R₁ and R₁' are both selected from the group consisting of phenyl and methoxy-substituted phenyl, in particular phenyl, para-methoxy-substituted phenyl and ortho,para-dimethoxy-substituted phenyl; or
(f) at least one of R₂ and R₂' represents an n-butyl group while the other one of R₂ and R₂' may represent an n-butyl group or a methyl group, and wherein R₁ and R₁' both represent a phenyl group.

11. Method as defined in any one of preceding claims 1 to 10, wherein R₁ and R₁' are the same and/or R₂ and R₂' are the same.

12. Method as defined in any one of the preceding claims 1 to 11, wherein a hydrogen source is used, which is selected from water and the group of compounds **characterized by** the general formula R₇R₈CH-CH₂-Br, wherein R₇ and R₈ are independently selected from hydrogen, a linear or branched C₁₋₂₀ alkyl group optionally substituted with a radical selected from hydroxyl and C₁₋₂₀ alkyloxy, a C₃₋₈ cycloalkyl optionally substituted with a radical selected from hydroxyl and C₁₋₂₀ alkyloxy, and a C₆₋₂₀ aryl.

13. Method as defined in any one of the preceding claims, wherein the Ni(II) salt is used in an amount of 0.9 mol% to 11 mol% with respect to the starting material being 100 mol% and/or the compound of formula (I) is used in an amount of 0.9 mol% to 55 mol% with respect to the starting material being 100 mol% and/or wherein the reducing agent is advantageously used in an amount of 100 mol% to 550 mol% with respect to the starting material being 100 mol%.

14. Method as defined in any one of the preceding claims, wherein the reaction is carried out at a temperature in the range of from 0 to 90°C and preferably in the range of from 5 to 85°C if the starting material is a compound with a leaving group X other than hydrogen or in the range of from 45 to 90°C if the starting material is a compound wherein X represents hydrogen.

15. Method as defined in any one of the preceding claims, wherein the reaction is carried out in a solvent consisting of or containing a solvent selected from DMF (N,N-Dimethylformamide), N,N-diethylformamide, DMSO (dimethylsulfoxide), DMA (N,N-dimethylacetamide), NMP (N-methylpyrrolidine), N,N-dibutylformamide and 1-formylpyrrolidine.
